# EUROPEAN PATENT SPECIFICATION

(11) **EP 1 838 850 B1**
(45) Date of publication and mention of the grant of the patent: **28.07.2010**
(21) Application number: 06703800.0
(22) Date of filing: 18.01.2006
(51) Int. Cl.: C12N 15/00, C12N 1/00, C12P 13/04

(54) **A METHOD FOR PRODUCING AN L-AMINO ACID USING A BACTERIUM OF THE ENTEROBACTERIACEAE FAMILY HAVING A PATHWAY OF GLYCOGEN BIOSYNTHESIS DISRUPTED**
METHODE ZUR HERSTELLUNG EINER L-AMINOSÄURE DURCH VERWENDUNG EINES BAKTERIUMS DER ENTEROBACTERIACEAE FAMILIE DESSEN GLYCOGEN BIOSYNTHESE PATHWAY UNTERBROCHEN IST
PROCEDE DE PRODUCTION D'ACIDE L-AMINE AU MOYEN D'UNE BACTERIE DE LA FAMILLE DES ENTEROBACTERIACEAE POSSEDANT UN TRAJET DE BIOSYNTHESE DE GLYCOGENE INTERROMPU

(30) Priority: 19.01.2005 RU 2005101110
(43) Date of publication of application: 03.10.2007
(73) Proprietor: Ajinomoto Co., Inc., Tokyo 104-8315 (JP)
(72) Inventor: RYBAK, Konstantin, Vyacheslavovich, Moscow 117149 (RU); SLIVINSKAYA, Ekaterina, Aleksandrovna, Moscow 103055 (RU); VOROSHILOVA, Elvira, Borisovna, Moscow 117648 (RU); KOZLOV, Yury, Ivanovich, Moscow 117574 (RU)
(74) Representative: HOFFMANN EITLE
(86) International application number: PCT/JP2006/301089
(87) International publication number: WO 2006/078050

(56) References cited:
- WO-A-95/04074
- TATARKO MATTHEW ET AL: "Disruption of a global regulatory gene to enhance central carbon flux into phenylalanine biosynthesis in Escherichia coli" CURRENT MICROBIOLOGY, vol. 43, no. 1, July 2001 (2001-07), pages 26-32, XP002398714 ISSN: 0343-8651
- SUZUKI KAZUSHI ET AL: "Regulatory circuitry of the CsrA/CsrB and BarA/UvrY systems of Escherichia coli" JOURNAL OF BACTERIOLOGY, vol. 184, no. 18, September 2002 (2002-09), pages 5130-5140, XP002398715 ISSN: 0021-9193
- WHITE D ET AL: "Phylogenetic distribution of the global regulatory gene csrA among eubacteria" GENE: AN INTERNATIONAL JOURNAL ON GENES AND GENOMES, ELSEVIER, AMSTERDAM, NL, vol. 182, no. 1-2, 5 December 1996 (1996-12-05), pages 221-223, XP004071956 ISSN: 0378-1119

## Description

### Technical Field

The present invention relates to the microbiological industry, and specifically to a method for producing an L-amino acid using a bacterium of the *Enterobacteriaceae* family, wherein the glycogen biosynthesis pathway has been disrupted.

### Background Art

Glycogen represents the major form of stored carbon for *Escherichia coli* and many other prokaryotes, and provides a readily metabolized substrate for maintenance energy. Glycogen accumulation in *Escherichia coli* is inversely related to the growth rate and occurs most actively when cells enter the stationary phase. The levels of the three biosynthetic enzymes undergo corresponding changes under these conditions, suggesting that genetic control of enzyme biosynthesis may account for at least part of the regulation (Preiss, J., Annu. Rev. Microbiol. 38,419-458 (1984)). In *Escherichia coli,* the structural genes for glycogen biosynthesis are clustered on adjacent operons - *glgBX* and *glgCAP.* Interestingly, the glycogen biosynthetic (*glgCA*) and degradative (*glgP*) genes are localized together in a cluster, possibly to facilitate the regulation of these systems *in vivo* (Romeo, T., Gene. 70(2), 363-76 (1988)). The *glg*C gene is the structural gene for glucose-1-phosphate adenylyltransferase. Synonyms for glucose-1-phosphate adenylyltransferase are ADP-glucose synthase, ADP-glucose pyrophosphorylase, ADP: a-D-glucose-1-phosphate adenylyltransferase, GlgC protein.

Glucose-1-phosphate adenylyltransferase (EC 2.7.7.27) is an allosteric enzyme in the glycogen biosynthetic pathway of eubacteria. Among the enteric bacteria, glucose-1-phosphate adenylyltransferase is regulated by glycolytic intermediates with fructose 1,6-biphosphate as the activator and AMP, ADP, and Pᵢ as inhibitors. The enzyme catalyzes the synthesis of ADP glucose and PPᵢ from glucose 1-phosphate and ATP. This reaction is the first unique step in bacterial glycogen biosynthesis.

It is known that the carbon storage regulatory system of *Escherichia coli* controls the expression of genes involved in carbohydrate metabolism and cell motility. CsrA binding to *glgCAP* transcripts inhibits glycogen metabolism by promoting *glgCAP* mRNA decay. CsrB RNA functions as an antagonist of CsrA by sequestering this protein and preventing its action (Baker, C.S. et al, Mol. Microbiol., 44(6), 1599-610 (2002)).

The *glgCAP* operon is under the positive control of cyclic AMP (cAMP) and the cAMP receptor protein (CRP). Both the *cya* gene encoding adenylate cyclase (EC 4.6.1.1) and the *crp* gene encoding CRP are required for optimal synthesis of glycogen (Fletterick, R.J. and Madsen, N.B., Annu. Rev. Biochem., 49, 31-61 (1980)). CRP binds to a site located upstream of the *glgC* gene. Glycogen synthesis in *E. coli* is also positively regulated by ppGpp, which stimulates the transcription of the *glgCAP* operon (Preiss. J., and Romeo, T., Prog. Nucleic Acid Res. Mol. Biol. 47,299-329 (1994)).

By using a mini-Mu random chromosomal library and screening for glycogen overproduction, a novel gene (*glgS*) involved in glycogen synthesis was identified (Hengge-Aronis, R. and Fischer, D., Mol Microbiol. 6, 14, 1877-86 (1992)). It was also shown that the *Escherichia coli* protein GIgS is up-regulated in response to starvation stress and its overexpression was shown to stimulate glycogen synthesis (Kozlov, G. et al, BMC Biol., 2, 1,10(2004)).

The initial substrate for glycolysis biosynthesis is glucose-1-P, obtained from glucose-6-P, so said pathway competes with glycolysis for glucose-6-P. But currently, there have been no reports of inactivating the *glgBX* and/or *glgCAP* operons or inactivating the *glgS* gene for producing L-amino acids.

### Disclosure of the Invention

Objects of the present invention include enhancing the productivity of L-amino acid producing strains and providing a method for producing an L-amino acid using these strains.

The above objects were achieved by finding that inactivating the *glgBX* and/or *glgCAP* operons can enhance production of L-amino acids, such as L-threonine, or L-lysine.

The present invention uses a bacterium of the *Enterobacteriaceae* family having an increased ability to produce amino acids, such as L-threonine, or L-lysine.

It is an object of the present invention to use an L-amino acid-producing bacterium of the *Enterobacteriaceae* family, wherein the bacterium has been modified so that the glycogen biosynthesis pathway is disrupted.

It is a further object of the present invention to use the bacterium as described above, wherein the glycogen biosynthesis pathway is disrupted by attenuation of expression of the *glgBX* and/or *glgCAP* operons.

It is a further object of the present invention to use the bacterium as described above, wherein the glycogen biosynthesis pathway is disrupted by inactivation of the *glgBX* and/or *glgCAP* operons.

It is a further object of the present invention to use the bacterium as described above, wherein the inactivation of the *glgBX* and/or *glgCAP* operons is performed by deletion of at least a gene selected from a group consisting of *glgB, glgX, glgC* and *glgA, glgP,* and combinations thereof.

It is a further object of the present invention to use the bacterium as described above, wherein the glycogen biosynthesis pathway is disrupted by attenuation of expression of the *gigS* gene.

It is a further object of the present invention to use the bacterium as described above, wherein the glycogen biosynthesis pathway is disrupted by inactivation of the *gigS* gene.

It is a further object of the present invention to use the bacterium as described above, wherein the bacterium belongs to the genus *Escherichia*.

It is a further object of the present invention to use the bacterium as described above, wherein the bacterium belongs to the genus *Pantoea.* It is a further object of the present invention to use the bacterium as described above, wherein said L-amino acid is selected from the group consisting of L-threonine, and L-lysine.

It is a further object of the present invention to provide a method for producing an L-amino acid comprising:
- cultivating the bacterium as described above in a medium to produce and excrete L-amino acid into the medium, and
- collecting said L-amino acid from the medium.

It is a further object of the present invention to provide the method as described above, wherein said non-aromatic L-amino acid is selected from the group consisting of L-threonine, and L-lysine.

### Brief Description of Drawings

Figure 1 shows the relative positions of primers glgCL and glgCR on plasmid pACYC184, which was used for amplification of the cat gene.
Figure 2 shows the construction of the chromosomal DNA fragment containing the inactivated *glgC* gene.
Figure 3 shows the construction of the chromosomal DNA fragment containing the inactivated *glgBX* and *glgCAP* operons.

### Detailed Description of the Preferred Embodiments

The present invention is described in detail below.

### 1. Bacterium for use in the present invention

The bacterium for use in a method of the present invention is an L-amino acid-producing bacterium of the *Enterobacteriaceae* family, wherein the bacterium has been modified so that the glycogen biosynthesis pathway is disrupted.

In the present invention, "L-amino acid-producing bacterium" means a bacterium which has an ability to produce and excrete L-amino acid into a medium, when the bacterium is cultured in the medium.

The term "L-amino acid-producing bacterium" as used herein also means a bacterium which is able to produce and cause accumulation of an L-amino acid in a culture medium in an amount larger than a wild-type or parental strain of *E. coli,* such as *E. coli* K-12, and preferably means that the microorganism is able to cause accumulation in a medium of an amount not less than 0.5 g/L, more preferably not less than 1.0 g/L, of the target L-amino acid. The term "L-amino acids" includes L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, and L-valine.

The term "aromatic L-amino acid" comprises L-phenylalanine, L-tyrosine, and L-tryptophan. The term "non-aromatic L-amino acid" comprises L-threonine, L-lysine, L-cysteine, L-methionine, L-leucine, L-isoleucine, L-valine, L-histidine, L-glycine, L-serine, L-alanine, L-asparagine, L-aspartic acid, L-glutamine, L-glutamic acid, L-proline, and L-arginine. L-threonine, L-lysine, L-cysteine, L-leucine, L-histidine, L-glutamic acid, L-phenylalanine. L-tryptophan, L-proline and L-arginine are particularly preferred.

The *Enterobacteriaceae* family includes bacteria belonging to the genera *Escherichia, Enterobacter, Erwinia, Klebsiella, Pantoea, Photorhabdus, Providencia, Salmonella, Serratia, Shigella, Morganella Yersinia,* etc.. Specifically, those classified into the *Enterobacteriaceae* according to the taxonomy used in the NCBI (National Center for Biotechnology Information) database (http://www.ncbi.nlm.nih.gov/htbinpostlTaxonomy/wgetorg?mode=Tree&id=1236&lvl=3& keep=1&srchmode=1&unlock) can be used. A bacterium belonging to the genus of *Escherichia* or *Pantoea* is preferred.

The phrase "a bacterium belonging to the genus *Escherichia"* means that the bacterium is classified into the genus *Escherichia* according to the classification known to a person skilled in the art of microbiology. Examples of a bacterium belonging to the genus *Escherichia* as used in the present invention include, but are not limited to, *Escherichia coli* (*E. coli*).

The bacterium belonging to the genus *Escherichia* that can be used in the present invention is not particularly limited, however for example, bacteria described by Neidhardt, F.C. et al. (*Escherichia coli* and *Salmonella typhimurium*, American Society for Microbiology, Washington D.C., 1208, Table 1) are encompassed by the present invention.

The phrase "a bacterium belonging to the genus *Pantoea*" means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii* or the like on the basis of nucleotide sequence analysis of 16S rRNA, etc. (Int. J. Syst. Bacteriol., 43, 162-173 (1993)). Such strains are encompassed by "bacteria belonging to the genus *Pantoea*".

The phrase "bacterium has been modified to have the glycogen biosynthesis pathway disrupted" means that the bacterium has been modified in such a way that the modified bacterium has a reduced ability to synthesize glycogen as compared with an unmodified bacterium or is unable to synthesize and accumulate glycogen. Disruption of glycogen biosynthetic pathway may be performed by attenuation of expression of genes encoding enzymes involved in glycogen biosynthesis, such as GlgB, GlgX, GlgC, GlgA, GlgP, and GlgS, and is preferably performed by inactivation of said genes.

The phrase "attenuation of expression of the *glgBX* and/or *glgCAP* operons" or "attenuation of expression of the *glgS* gene"means that the target operon or gene is modified in such a way that the modified operon or gene encodes a mutant protein(s) which has (have) a decreased activity. The phrase "inactivation of the *glgBX* and/or *glgCAP* operons" or "inactivation of the *glgS* gene" means that such modified operon or gene encodes a completely inactive protein(s). It is also possible that the modified DNA region is unable to naturally express the gene(s) due to the deletion of a part of the gene(s), the shifting of the reading frame of the gene(s), the introduction of missense/nonsense mutation(s), or the modification of an adjacent region of the gene(s), including sequences controlling gene expression, such as promoter(s), enhancer(s), attenuator(s), ribosome-binding site(s), etc..

The level of gene expression can be estimated by measuring the amount of mRNA transcribed from the gene using various known methods including Northern blotting, quantitative RT-PCR, and the like. The amount of the protein encoded by the gene can be measured by known methods including SDS-PAGE followed by immunoblotting assay (Western blotting analysis) and the like.

The *glgC* gene encodes glucose-1-phosphate adenylyltransferase (synonym - B3430). The *glgC* gene (gi: 16131304; nucleotides complementary to nucleotides 3566056 to 3567351 in the GenBank accession number NC_000913.2; SEQ ID NO: 1) is located between the *glgA* and *glgX* genes on the chromosome of *E. coli* K-12. The nucleotide sequence of the *glgC* gene and the amino acid sequence of the glucose-1-phosphate adenylyltransferase encoded by the *glgC* gene are shown in SEQ ID NO: and SEQ ID NO:2, respectively.

The *glgA* gene encodes a subunit of glycogen synthase (synonym - B3429). The *glgA* gene (nucleotide positions: 3,566,056 to 3,564,623; GenBank accession no. NC_000913.2; gi: 49175990) is located between the *glgP* and *glgC* genes on the chromosome of *E*. *coli* K-12. The nucleotide sequence of the *glgA* gene and the amino acid sequence of the subunit of glycogen synthase encoded by the *glgA* gene are shown in SEQ ID NO:11 and SEQ ID NO:12, respectively.

The *glgP* gene encodes a subunit of glycogen phosphorylase / glycogen maltotetraose phosphorylase (synonyms - B3428, Gigs). The *glgP* gene (nucleotide positions: 3,564,604 to 3,562,157; GenBank accession no. NC_000913.2; gi: 49175990) is located between the *yzgL* and *glgA* genes on the chromosome of *E. coli* K-12. The nucleotide sequence of the *glgP* gene and the amino acid sequence of the subunit of glycogen phosphorylase / glycogen-maltotetraose phosphorylase encoded by the *glgP* gene are shown in SEQ ID NO:13 and SEQ ID NO:14, respectively.

The *glgX* gene encodes glycogen phosphorylase-limit dextrin α-1,6-glucohydrolase (synonyms - B3431, GlyX). The *glgX* gene (nucleotide positions: 3,569,342 to 3,567,369; GenBank accession no. NC_000913.2; gi: 49175990) is located between the *glgC* and *glgB* genes on the chromosome of *E. coli* K-12. The nucleotide sequence of the *glgX* gene and the amino acid sequence of the glycogen phosphorylase-limit dextrin α-1,6-glucohydrolase encoded by the *glgX* gene are shown in SEQ ID NO:15 and SEQ ID NO:16, respectively.

The *glgB* gene encodes a glycogen-branching enzyme (synonym - B3432). The *glgB* gene (nucleotide positions: 3,571,525 to 3,569,339; GenBank accession no. NC_000913.2; gi: 49175990) is located between the *glgX* and *asd* genes on the chromosome of *E. coli* K-12. The nucleotide sequence of the *glgB* gene and the amino acid sequence of the glycogen-branching enzyme encoded by the *glgB* gene are shown in SEQ ID NO:17 and SEQ ID NO:18, respectively.

The *glgS* gene encodes a *rpoS*-dependent protein of glycogen biosynthesis (synonym - B3049). The *glgS* gene (nucleotide positions: 3,189,961 to 3,189,761; GenBank accession no. NC_000913.2; gi: 49175990) is located between the *yqiI* and *yqiJ* ORFs on the chromosome of *E*. *coli* K-12. The nucleotide sequence of the *glgS* gene and the amino acid sequence of the *rpoS*-dependent protein of glycogen biosynthesis encoded by the *glgS* gene are shown in SEQ ID NO:19 and SEQ ID NO:20, respectively.

Since there may be some differences in DNA sequences between the genera or strains of the *Enterobacteriaceae* family, the *glgC* gene to be deleted on the chromosome is not limited to the gene shown in SEQ ID No:1 but may include a homologous gene of SEQ ID No: 1. Therefore, the protein variant encoded by the *glgC* gene may have a homology of not less than 80%, preferably not less than 90%, and most preferably not less than 95%, with respect to the entire amino acid sequence encoded shown in SEQ ID NO. 2, as long as the activity of glucose-1-phosphate adenylyltransferase prior to inactivation is maintained.

Moreover, the *glgC* gene may be a variant which hybridizes under stringent conditions with the nucleotide sequence shown in SEQ ID NO:1, or a probe which can be prepared from the nucleotide sequence, provided that it encodes a 1-phosphate adenylyltransferase prior to inactivation. "Stringent conditions" include those under which a specific hybrid, for example, a hybrid having homology of not less than 60%, preferably not less than70%, more preferably not less than 80%, and still more preferably not less than 90%, and most preferably not less than 95% is formed and a non-specific hybrid, for example, a hybrid having homology lower than the above is not formed. For example, stringent conditions are exemplified by washing one time, preferably two or three times at a salt concentration corresponding to 1 X SSC, 0.1% SDS, preferably 0.1 X SSC, 0.1% SDS at 60°C. The length of the probe may be suitably selected depending on the hybridization conditions, and is usually 100 bp to 1 kbp.

The similar expression as described above for the *glgC* gene can be applied for the variants of other genes of the *glgBX* and *glgCAP* operons.

Inactivation of the gene can be performed by conventional methods, such as mutagenesis treatment using UV irradiation or nitrosoguanidine (N-methyl-N'-nitro-N-nitrosoguanidine) treatment, site-directed mutagenesis, gene disruption using homologous recombination, or/and insertion-deletion mutagenesis (Yu, D. et al., Proc. Natl. Acad. Sci. USA, 2000, 97:12: 5978-83) and (Datsenko, K.A. and Wanner, B.L., Proc. Natl. Acad. Sci: USA, 2000, 97:12: 6640-45) also called "Red-driven integration".

Activity of glucose-1-phosphate adenylyltransferase encoded by the *glgC* gene can be measured by the method described by, for example, Haugen, T.H. et al (J. Biol. Chem. 251 (24), 7880-5 (1976)). So, the decreasing or absent activity of glucose-1-phosphate adenylyltransferase in the bacterium for use in a method according the present invention can be determined when compared to the parent unmodified bacterium.

Activity of glycogen synthase encoded by the *glgA* gene can be measured by the method described by, for example, Fox, J. et al (Methods Enzymol., 28, 539-544 (1972)). So, the decreasing or absent activity of glycogen synthase in the bacterium for use in a method according the present invention can be determined when compared to the parent unmodified bacterium.

Activity of glycogen phosphorylase / glycogen-maltotetraose phosphorylase encoded by the *glgP* gene can be measured by the method described by, for example, Graves, D.J. and Wang, J.H. (The Enzymes, 7 (3rd ed.), 435-482 (1972)). So, the decreasing or absent activity of glycogen phosphorylase / glycogen-maltotetraose phosphorylase in the bacterium for use in a method according the present invention can be determined when compared to the parent unmodified bacterium.

Activity of glycogen phosphorylase-limit dextrin α-1,6-glucohydrolase encoded by the *glgX* gene can be measured by the method described by, for example, Jeanningros, R. et al (Biochim Biophys Acta, 438(1), 186-199 (1976)). So, the decreasing or absent activity of glycogen phosphorylase-limit dextrin α-1.6-glucohydrolase in the bacterium for use in a method according the present invention can be determined when compared to the parent unmodified bacterium.

Activity of glycogen-branching enzyme encoded by the *glgB* gene can be measured by the method described by, for example, Illingworth, B.B. and Brown, D.H. (Methods Enzymol., 8, 395-403 (1966)). So, the decreasing or absent activity of glycogen-branching enzyme in the bacterium for use in a method according the present invention can be determined when compared to the parent unmodified bacterium.

Activity of *poS*-dependent protein of glycogen biosynthesis encoded by the *glgS* gene can be detected by complementation of glgS-null mutation which inhibits glycogen synthesis as described by, for example, Hengge-Aronis, R, and Fischer, D. (Mol. Microbiol., 6, 14, 1877-1886 (1992)). So, the decreasing or absent activity of *rpoS* dependent protein of glycogen biosynthesis in the bacterium for use in a method according the present invention can be determined when compared to the parent unmodified bacterium.

Methods for preparation of plasmid DNA, digestion and ligation of DNA, transformation, selection of an oligonucleotide as a primer and the like may be ordinary methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

### L-amino acid-producing bacteria

As a bacterium for use in a method of the present invention, which is modified to inactivate the *glgBX* and/or *glgCAP* operons or the *glgS* gene, bacteria which are able to produce either L-threonine or L-lysine may be used.

The bacterium for use in a method of the present invention can be obtained by inactivating the *glgBX* and/or *glgCAP* operons or the *glgS* gene in a bacterium which inherently has the ability to produce L-amino acids. Alternatively, the bacterium for use in a method of the present invention can be obtained by imparting the ability to produce L-amino acids to a bacterium already having the *glgBX* and/or *glgCAP* operons or the *glgS* gene inactivated.

### L-threonine-producing bacteria

Examples of parent strains for deriving the L-threonine-producing bacteria for use in a method of the present invention include, but are not limited to, strains belonging to the genus *Escherichia,* such as *E. coli* TDH-6/pVIC40 (VKPM B-3996) (U.S. Patent No. 5,175, 107, U.S. Patent No. 5,705,371), *E. coli* NRRL-21593 (U.S. Patent No. 5.939,307), *E. coli* FERM Bop-3756 (U.S. Patent No. 5,474,918), *E. coli* FERM Bop-3519 and FERM BP-3520 (U.S. Patent No. 5,376,538), *E. coli* MG442 (Gusyatiner et al., Genetika (in Russian), 14, 947-956 (1978)), *E. coli* VL643 and VL2055 (EP 1149911 A), and the like.

The strain TDH-6 is deficient in the *thrC* gene, as well as being sucrose-assimilative, and the *ilvA* gene has a leaky mutation. This strain also has a mutation in the *rhtA* gene, which imparts resistance to high concentrations of threonine or homoserine. The strain B-3996 contains the plasmid pVIC40 which was obtained by inserting a *thrA*BC* operon which includes a mutant *thrA* gene into a RSF1010-derived vector. This mutant *thrA gene* encodes aspartokinase homoserine dehydrogenase I which has substantially desensitized feedback inhibition by threonine. The strain B-3996 was deposited on November 19, 1987 in the All-Union Scientific Center of Antibiotics (Nagatinskaya Street 3-A, 117105 Moscow, Russian Federation) under the accession number RIA 1867. The strain was also deposited in the Russian National Collection of Industrial Microorganisms (VKPM) (Russia, 117545 Moscow , 1 Dorozhny proezd,. 1) on April 7, 1987 under the accession number B-3996.

Preferably, the bacterium of the present invention is additionally modified to enhance expression of one or more of the following genes:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase);

The *thrA* gene which encodes aspartokinase homoserine dehydrogenase I of *Escherichia coli* has been elucidated (nucleotide positions 337 to 2799, GenBank accession NC_000913.2, gi: 49175990). The *thrA* gene is located between the *thrL* and *thrB* genes on the chromosome of *E. coli* K-12. The *thrB* gene which encodes homoserine kinase of *Escherichia coli* has been elucidated (nucleotide positions 2801 to 3733, GenBank accession NC_000913.2, gi: 49175990). The *thrB* gene is located between the *thrA* and *thrC* genes on the chromosome of *E. coli* K-12. The *thrC* gene which encodes threonine synthase of *Escherichia coli* has been elucidated (nucleotide positions 3734 to 5020, GenBank accession NC_000913.2, gi: 49175990). The *thrC* gene is located between the *thrB* gene and the *yaaX* open reading frame on the chromosome of *E. coli* K-12. All three genes functions as a single threonine operon.

A mutant *thrA* gene which codes for aspartokinase homoserine dehydrogenase I resistant to feed back inhibition by threonine, as well as, the *thrB* and *thrC* genes can be obtained as one operon from well-known plasmid pVIC40 which is presented in the threonine producing *E. coli* strain VKPM B-3996. Plasmid pVIC40 is described in detail in U.S. Patent No. 5,705,371.

The *rhtA* gene exists at 18 min on the *E. coli* chromosome close to the *glnHPQ* operon, which encodes components of the glutamine transport system. The *rhtA* gene is identical to ORF1 (*ybiF* gene, nucleotide positions 764 to 1651, GenBank accession number AAA218541, gi:440181) and located between the *pexB* and *ompX* genes. The unit expressing a protein encoded by the ORF1 has been designated the *rhtA* gene (rht: resistance to homoserine and threonine). Also, it was revealed that the rhtA23 mutation is an A-for-G substitution at position -1 with respect to the ATG start codon (ABSTRACTS of the 17^{th} International Congress of Biochemistry and Molecular Biology in conjugation with Annual Meeting of the American Society for Biochemistry and Molecular Biology, San Francisco, California August 24-29, 1997, abstract No. 457, EP 1013765 A).

The *asd* gene of *E.coli* has already been elucidated (nucleotide positions 3572511 to 3571408, GenBank accession NC_000913.1, gi:16131307), and can be obtained by PCR (polymerase chain reaction; refer to White, T.J. et al., Trends Genet., 5, 185 (1989)) utilizing primers prepared based on the nucleotide sequence of the gene. The *asd* genes of other microorganisms can be obtained in a similar manner.

Also, the *aspC* gene of *E.coli* has already been elucidated (nucleotide positions 983742 to 984932, GenBank accession NC_000913.1, gi:16128895), and can be obtained by PCR. The *aspC* genes of other microorganisms can be obtained in a similar manner.

### L-lysine-producing bacteria

Examples of L-lysine-producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include, but are not limited to, oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611*.* In these microorganisms, feedback inhibition of aspartokinase by L-lysine is desensitized.

The strain WC196 may be used as an L-lysine producing bacterium of *Escherichia coli.* This bacterial strain was bred by conferring AEC resistance to the strain W3110, which was derived from *Escherichia coli* K-12. The resulting strain was designated *Escherichia coli* AJ13069 strain and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of parent strains for deriving L-lysine-producing bacteria for use in a method of the present invention also include strains in which expression of one or more genes encoding an L-lysine biosynthetic enzyme are enhanced. Examples of the enzymes involved in L-lysine biosynthesis include, but are not limited to, dihydrodipicolinate synthase (*dapA*), aspartokinase (*lvsC*), dihydrodipicolinate reductase (*dapB*), diaminopimelate decarboxylase (*lysA*), diaminopimelate dehydrogenase (*ddh*) (U.S. Patent No. 6,040,160), phosphoenolpyrvate carboxylase (*ppc*), aspartate semialdehyde dehydrogenease (*asd*), nicotinamide adenine dinucleotide transhydrogenase (*pntAB*), and aspartase (*aspA*) (EP 1253195 A).

Examples of parent strains for deriving L-lysine-producing bacteria for use in a method of the present invention also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase and lysine decarboxylase (U.S. Patent No. 5,827,698).

### 2. Method of the present invention

The method of the present invention is a method for producing an L-amino acid comprising cultivating the bacterium as described above in a culture medium to produce and excrete the L-amino acid into the medium, and collecting the L-amino acid from the medium.

In the present invention, the cultivation, collection, and purification of an L-amino acid from the medium and the like may be performed in a manner similar to conventional fermentation methods wherein an amino acid is produced using a bacterium.

A medium used for culture may be either a synthetic or natural medium, so long as the medium includes a carbon source and a nitrogen source and minerals and, if necessary, appropriate amounts of nutrients which the bacterium requires for growth. The carbon source may include various carbohydrates such as glucose and sucrose, and various organic acids. Depending on the mode of assimilation of the used microorganism, alcohol, including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used. As vitamins, thiamine, yeast extract, and the like, can be used.

The cultivation is preferably performed under aerobic conditions, such as a shaking culture, and a stirring culture with aeration, at a temperature of 20 to 40°C, preferably 30 to 38 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of the target L-amino acid in the liquid medium.

After cultivation, solids such as cells can be removed from the liquid medium by centrifugation or membrane filtration, and then the L-amino acid can be collected and purified by ion-exchange, concentration and/or crystallization methods.

### Examples

The present invention will be more concretely explained below with reference to the following non-limiting Examples.

### Example 1. Construction of a strain with an inactivated glgC gene

### 1. Deletion of the glgC gene

A strain having deletion of the *glgC* gene can be constructed by the method initially developed by Datsenko, K.A. and Wanner, B.L. (Proc. Natl. Acad. Sci. USA, 2000, 97(12), 6640-6645) called "Red-driven integration". According to this procedure, the PCR primers glgCL (SEQ ID NO: 3) and glgCR (SEQ ID NO: 4) complementary to both the region adjacent to the *glgC* gene and the gene conferring antibiotic resistance in the template plasmid can be constructed. The plasmid pACYC184 (NBL Gene Sciences Ltd., UK) (GenBank/EMBL accession number X06403) can be used as a template in PCR reaction. Conditions for PCR can be as follows: denaturation step at 95°C for 3 min; profile for two first cycles: 1 min at 95°C, 30 sec at 50°C, 40 sec at 72°C; profile for the last 25 cycles: 30 sec at 95°C, 30 sec at 54°C, 40 sec at 72°C; final step: 5 min at 72°C.

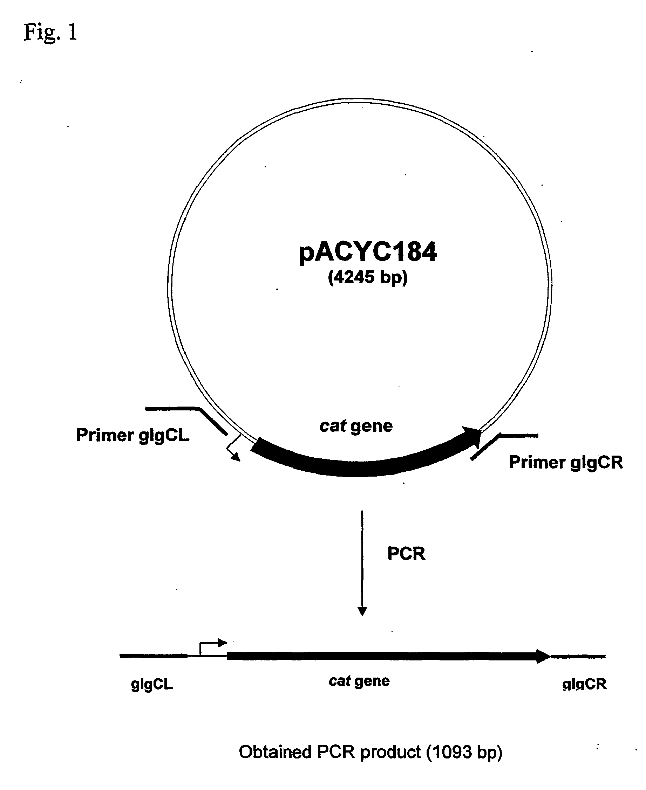

A 1093-bp PCR product (Fig. 1) can be obtained and purified in agarose gel and can be used for electroporation of *E. coli* MG1655 (ATCC 700926), which contains the plasmid pKD46 having temperature-sensitive replication. The plasmid pKD46 (Datsenko, K.A. and Wanner, B.L., Proc. Natl. Acad. Sci. USA, 2000, 97:12:6640-45) includes a 2,154 nucleotide (31088-33241) DNA fragment of phage λ (GenBank accession No. J02459), and contains genes of the λ Red homologous recombination system (γ, β, exo genes) under the control of the arabinose-inducible P_{araB} promoter. The plasmid pKD46 is necessary for integration of the PCR product into the chromosome of strain MG1655.

Electrocompetent cells can be prepared as follows: a night culture of *E. coli* MG1655 can be grown at 30°C in LB medium supplemented with ampicillin (100 mg/l) and then diluted 100-fold with 5 ml of SOB medium (Sambrook et al, "Molecular Cloning A Laboratory Manual, Second Edition", Cold Spring Harbor Laboratory Press (1989)) containing ampicillin and L-arabinose (1 mM). The obtained culture can be grown with aeration at 30°C to an OD₆₀₀ of ≈0.6 and then can be made electrocompetent by concentrating 100-fold and washing three times with ice-cold deionized H₂O. Electroporation can be performed using 70 µl of cells and ≈100 ng of the PCR product. Cells after electroporation can be incubated with 1 ml of SOC medium (Sambrook et al, "Molecular Cloning A Laboratory Manual, Second Edition", Cold Spring Harbor Laboratory Press (1989)) at 37°C for 2.5 hours and after that can be plated onto L-agar and grown at 37°C to select Cm^{R} recombinants. Then, to eliminate the pKD46 plasmid, 2 passages on L-agar with Cm at 42°C can be performed and the obtained colonies can be tested for sensitivity to ampicillin.

### 2. Verification of the glgC gene deletion by PCR

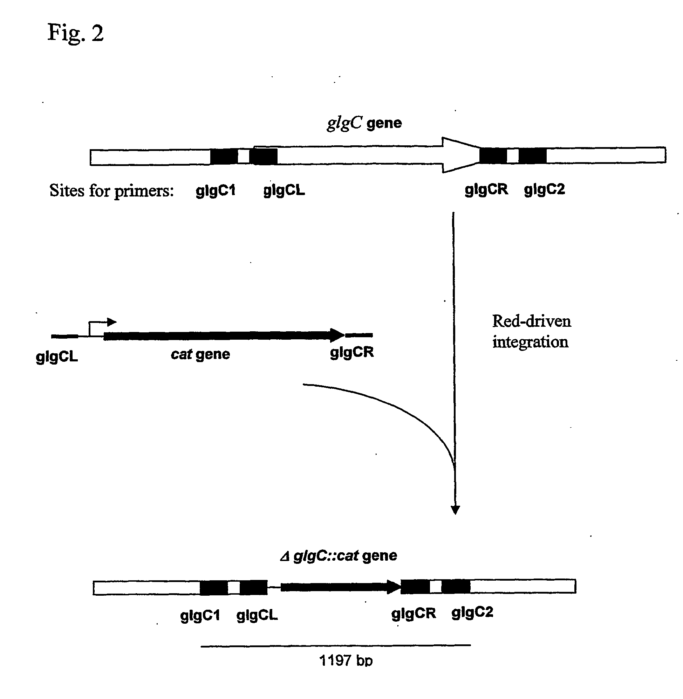

The mutants, containing the deletion of the *glgC* gene, marked with Cm resistance gene, can be verified by PCR. Locus-specific primers glgC1 (SEQ ID NO: 5) and glgC2 (SEQ ID NO: 6) can be used in PCR for verification. Conditions for PCR verification can be as follows: denaturation step at 94°C for 3 min; profile for the 30 cycles: 30 sec at 94°C, 30 sec at 54°C, 1 min at 72°C; final step: 7 min at 72°C. The PCR product, which can be obtained in the reaction with the parental strain MG1655 glgC⁺ as a template, should be 1471 bp in length. The PCR product, which can be obtained in the reaction with the mutant MG1655 ΔglgC::cat strain as a template, should be 1197 bp in length (Fig.2).

### Example 2. Production of L-threonine by E. coli B-3996-ΔglgC

The deletion of the *glgC* gene in the chromosome of *E. coli* L-threonine-producing strain B-3996 (VKPM B-3996) can be performed by an ordinary well-known method as described above to obtain strain·B-3996-ΔglgC.

Both *E. coli* strains, B-3996 and B-3996-ΔglgC, can be grown for 18-24 hours at 37°C on L-agar plates. Then these strains can be cultivated under the same conditions as described above.

### Example 3. Production of L-lysine by E. coli AJ11442-ΔglgC

The deletion of the *glgC* gene in the chromosome of the *E. coli* L-lysine-producing strain AJ11442 (FERM Bop-1543, NRRL B-12185) can be performed by an ordinary well-known method as described above to obtain strain AJ11442-AglgC. The strain AJ11442 was deposited at the Fermentation Research Institute, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on May 1, 1981 and received an accession number of FERM P-5084. Then, it was converted to an international deposit under the provisions of the Budapest Treaty on October 29, 1987, and received an accession number of FERM BP-1543.

Both *E. coli* strains, AJ11442 and AJ11442-ΔglgC, can be grown for 18-24 hours at 37°C on L-agar plates. Then these strains can be cultivated under the same conditions as described above.

### Example 4. Construction of a strain with the glgBX and glgCAP operons inactivated

### 1. Deletion of the glgBX and glgCAP operons

A strain having deletion of the *glgBX* and *glgCAP* operons was constructed by the method initially developed by Datsenko, K.A. and Wanner, B.L. (Proc. Natl. Acad. Sci. USA, 2000, 97(12), 6640-6645) called "Red-driven integration". According to this procedure, the PCR primers glgBXCAPL (SEQ ID NO: 7) and glgBXCAPR (SEQ ID NO: 8) complementary to both the region adjacent to the *glgBX* and *glgCAP* operons and the gene conferring antibiotic resistance in the template plasmid were constructed. The plasmid pACYC184 (NBL Gene Sciences Ltd., UK) (GenBank/EMBL accession number X06403) was used as a template in PCR. Conditions for PCR were described in detail in Example 1.

A 1152-bp PCR product purified in agarose gel was used for electroporation of *E. coli* MG1655 (ATCC 700926) containing the plasmid pKD46 having temperature-sensitive replication, as described in Example 1.

### 2. Verification of the deletion of the glgBX and glgCAP operons by PCR

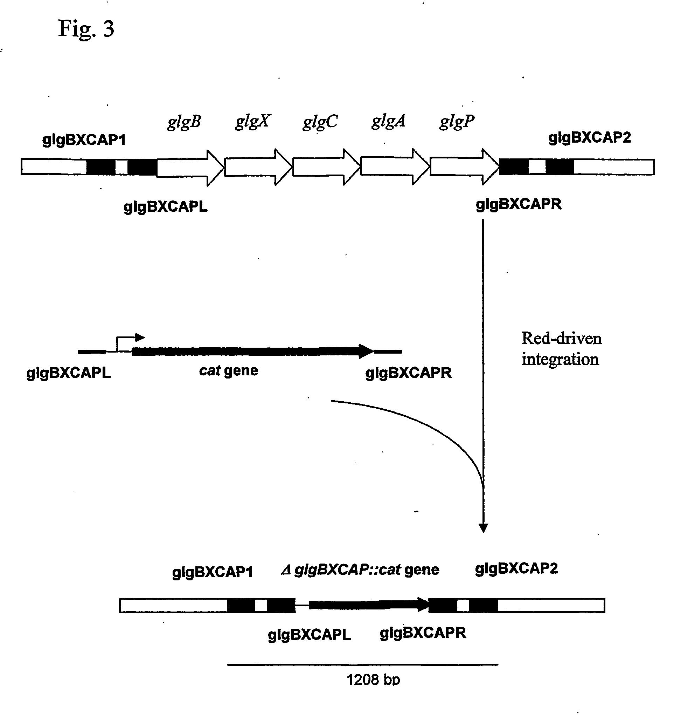

The mutants containing the deletion of the *glgBX* and *glgC4P* operons and marked with Cm resistance gene were verified by PCR. Locus-specific primers glgBXCAP1 (SEQ ID NO: 9) and glgBXCAP2 (SEQ ID NO: 10) were used in PCR for the verification. Conditions for PCR verification were described in Example 1. The PCR product obtained in the reaction with the parental strain MG1655 glgBXCAP⁺ as a template was 9425 bp in length. The PCR product obtained in the reaction with the mutant strain MG1655 ΔglgBXCAP::cat as a template was 1208 bp in length (Fig. 3).

### Example 5. Production of L-threonine by E. coli strain B-3996-ΔglgBXCAP

To test the effect of inactivation of the *glgBX* and *glgCAP* operons on threonine production, DNA fragments from the chromosome of the above-described *E. coli* strain MG1655 ΔglgBXCAP::cat were transferred to the threonine-producing *E. coli* strain B-3996 by P1 transduction (Miller, J.H. Experiments in Molecular Genetics, Cold Spring Harbor Lab. Press, 1972, Plainview, NY).

Both *E. coli* strains, B-3996 and B-3996-ΔglgBXCAP, were grown for 18-24 hours at 37°C on L-agar plates. To obtain a seed culture, the strains were grown on a rotary shaker (250 rpm) at 32°C for 18 hours in 20x200-mm test tubes containing 2 ml of L-broth supplemented with 4% glucose. Then the fermentation medium was inoculated with 0.21 ml (10%) of seed material. The fermentation was performed in 2 ml of minimal medium for fermentation in 20x200-mm test tubes. Cells were grown for 65 hours at 32°C with shaking at 250 rpm.

After cultivation, the amount of L-threonine accumulated in the medium was determined by paper chromatography using the following mobile phase: butanol - acetic acid - water = 4 : 1 : 1 (v/v). A solution of ninhydrin (2%) in acetone was used as a visualizing reagent. A spot containing L-threonine was cut off, L-threonine was eluted with 0.5% water solution of CdCl₂, and the amount of L-threonine was estimated spectrophotometrically at 540 nm. The results of ten independent test-tube fermentations are shown in Table 1.

The composition of the fermentation medium (g/l) was as follows:

| | |
|---|---|
| Glucose | 80.0 |
| (NH₄)₂SO₄ | 22.0 |
| NaCl | 0.8 |
| KH₂PO₄ | 2.0 |
| MgSO₄·7H₂O | 0.8 |
| FeSO₄·7H₂O | 0.02 |
| MnS0₄·5H₂O | 0.02 |
| Thiamine HCl | 0.0002 |
| Yeast extract | 1.0 |
| CaCO₃ | 30.0 |

Glucose and magnesium sulfate were sterilized separately. CaCO₃ was sterilized by dry-heat at 180°C for 2 hours. The pH was adjusted to 7.0. The antibiotic was added to the medium after sterilization.

As follows from Table 1, B-3996-ΔglgBXCAP caused accumulation of a higher amount of L-threonine, as compared with B-3996.

### Example 6. Production of L-lysine by E. coli WC196-ΔglgBXCAP

To test the effect of inactivation of the *glgBX* and *glgCAP* operons on L-lysine production, DNA fragments from the chromosome of the above-described *E. coli* strain MG1655 ΔglgBXCAP::cat were transferred to the L-lysine producing *E. coli* strain WC196 (FERM BP-5252) by P1 transduction (Miller, J.H. Experiments in Molecular Genetics, Cold Spring Harbor Lab. Press, 1972, Plain view, NY).

To obtain a seed culture, both *E. coli* strains, WC196 and WC196-ΔglgBXCAP, were grown on a rotary shaker (250 rpm) at 32 °C for 18 hours in 20x200-mm test tubes containing 2 ml of medium diluted two times compare to the fermentation medium described below. Then the fermentation medium was inoculated with 0.21 ml (10%) of seed material. The fermentation was performed in 2 ml of minimal medium for fermentation in 20x200-mm test tubes. Cells were grown for 24 hours at 32 °C with shaking at 250 rpm.

After cultivation, the amount of L-lysine accumulated in the medium was determined by paper chromatography using the following mobile phase: butanol - acetic acid - water = 4:1:1 (v/v). A solution of ninhydrin (2%) in acetone was used as a visualizing reagent. A spot containing L-lysine was cut off, L-lysine was eluted with 0.5% water solution of CdCl₂, and the amount of L-lysine was estimated spectrophotometrically at 540 nm. The results of ten independent test-tube fermentations are shown in Table 2.

The composition of the fermentation medium (g/l) was as follows:

| | |
|---|---|
| Glucose | 40.0 |
| (NH₄)₂SO₄ | 24.0 |
| KH₂PO₄ | 1.0 |
| MgSO₄·7H₂O | 1.0 |
| FeSO₄·7H₂O | 0.01 |
| MnSO₄·5H₂O | 0.01 |
| Yeast extract | 2.0 |
| CaCO₃ | 30.0 |

Glucose, potassium phosphate and magnesium sulfate were sterilized separately. CaCO₃ was sterilized by dry-heat at 180°C for 2 hours. The pH was adjusted to 7.0.

As follows from Table 2, WC196-ΔglgBXCAP caused accumulation of a higher amount of L-lysine, as compared with WC196.

While the invention has been described in detail with reference to preferred embodiments thereof, it will be apparent to one skilled in the art that various changes can be made, and equivalents employed, without departing from the scope of the invention. All the cited references herein are incorporated as a part of this application by reference.

**Table 1.**

| Strain | OD₅₄₀ | Amount of L-threonine, g/l |
|---|---|---|
| B-3996 | 23.5 ± 0.3 | 27.5 ± 0.4 |
| B-3996-ΔglgBXCAP | 22.1 ± 0.2 | 28.2 ± 1.2 |

**Table 2.**

| Strain | OD₅₄₀ | Amount of L-lysine, g/l |
|---|---|---|
| WC196 | 26.2 ± 0.5 | 2.1 ± 0.1 |
| WC196-ΔglgBXCAP | 26.9 ± 0.3 | 2.4 ± 0.1 |

### Industrial Applicability

According to the present invention, production of an aromatic L-amino acid or a non-aromatic L-amino acid of a bacterium of the *Enterobacteriaceae* family can be enhanced.

## Claims

1. A method for producing L-amino acid comprising:
- cultivating an L-amino acid-producing bacterium of the *Enterobacteriaceae* family in a medium to produce and excrete said L-amino acid into the medium, and
- collecting said L-amino acid from the medium, wherein the bacterium has been modified so that the glgBX and glgCAP operons are disrupted, and wherein said L-amino acid is selected from the group consisting of L-threonine and L-lysine.

2. The method according to claim 1, wherein said glgBX and glgCAP operons are disrupted by attenuation of expression of the glgBX and glgCAP operons.

3. The method according to claim 1, wherein said glgBX and glgCAP operons are disrupted by inactivation of the glgBX and glgCAP operons.

4. The method according to claim 3, wherein the inactivation of the glgBX and glgCAP operons is performed by deletion of all of glgB, glgX, glgC, glgA, and glgP genes.

5. The method according to any one of claims 1 to 4, wherein said bacterium belongs to the genus Escherichia.

6. The method according to claim 5, wherein said bacterium is Escherichia coli.

7. The method according to any one of claims 1 to 4, wherein said bacterium belongs to the genus Pantoea.

## Patentansprüche

1. Verfahren zur Herstellung einer L-Aminosäure, umfassend:
- Kultivieren eines L-Aminosäure herstellenden Bakteriums der Enterobacteriaceae-Familie in einem Medium zur Herstellung und Ausscheidung der L-Aminosäure in das Medium, und
- Gewinnen der L-Aminosäure aus dem Medium,
wobei das Bakterium modifiziert worden ist, so dass das glgBX- und glgCAP-Operon unterbrochen ist, und wobei die L-Aminosäure ausgewählt ist aus der Gruppe, bestehend aus L-Threonin und L-Lysin.

2. Verfahren gemäß Anspruch 1, wobei das glgBX- und glgCAP-Operon durch Verringerung der Expression des glgBX- und glgCAP-Operons unterbrochen ist.

3. Verfahren gemäß Anspruch 1, wobei das glgBX- und glgCAP-Operon durch Inaktivierung des glgBX- und glgCAP-Operons unterbrochen ist.

4. Verfahren gemäß Anspruch 3, wobei die Inaktivierung des glgBX- und glgCAP-Operons durch Deletion aller der Gene glgB, glgX, glgC, glgA und glgP durchgeführt wird.

5. Verfahren gemäß einem der Ansprüche 1 bis 4, wobei das Bakterium zu der Gattung Escherichia gehört.

6. Verfahren gemäß Anspruch 5, wobei das Bakterium Escherichia coli ist.

7. Verfahren gemäß einem der Ansprüche 1 bis 4, wobei das Bakterium zu der Gattung Pantoea gehört.

## Revendications

1. Procédé pour produire un acide L-aminé comprenant les étapes consistant à:
- cultiver une bactérie produisant de l'acide L-aminé de la famille *Enterobacteriaceae* dans un milieu pour produire et excréter ledit acide L-aminé dans le milieu, et
- collecter ledit acide L-aminé à partir du milieu, dans lequel la bactérie a été modifiée de sorte que les opérons glgBX et glgCAP sont désunis, et dans lequel ledit acide L-aminé est sélectionné dans le groupe constitué par la L-thréonine et la L-lysine.

2. Procédé selon la revendication 1, dans lequel lesdits opérons glgBX et glgCAP sont désunis par atténuation d'expression des opérons glgBX et glgCAP.

3. Procédé selon la revendication 1, dans lequel lesdits opérons glgBX et glgCAP sont désunis par inactivation des opérons glgBX et glgCAP.

4. Procédé selon la revendication 3, dans lequel l'inactivation des opérons glgBX et glgCAP est effectuée par la délétion de tous les gènes glgB, glgX, glgC, glgA et glgP.

5. Procédé selon l'une quelconque des revendications 1 à 4, dans lequel ladite bactérie appartient au genre Escherichia.

6. Procédé selon la revendication 5, dans lequel ladite bactérie est Escherichia coli.

7. Procédé selon l'une quelconque des revendications 1 à 4, dans lequel ladite bactérie appartient au genre Pantoea.


### SEQUENCE LISTING

<110> Ajinomoto Co., Inc.
<120> A METHOD FOR PRODUCING AN L-AMINO ACID USING A BACTERIUM OF THE ENTEROBACTERIACEAE FAMILY HAVING A PATHWAY OF GLYCOGEN BIOSYNTHESIS DISRUPTED
<130> C440-C5323
<150> RU2005101110
   <151> 2005-01-19
<160> 20
<170> PatentIn version 3.1
<210> 1
   <211> 1296
   <212> DNA
   <213> Escherichia coli
<220>
   <221> CDS
   <222> (1)..(1296)
<400> 1
   atg gtt agt tta gag aag aac gat cac tta atg ttg gcg cgc cag ctg        48
   Met Val Ser Leu Glu Lys Asn Asp His Leu Met Leu Ala Arg Gln Leu
   1               5                   10                  15
   cca ttg aaa tct gtt gcc ctg ata ctg gcg gga gga cgt ggt acc cgc        96
   Pro Leu Lys Ser Val Ala Leu Ile Leu Ala Gly Gly Arg Gly Thr Arg
               20                  25                  30
   ctg aag gat tta acc aat aag cga gca aaa ccg gcc gta cac ttc ggc       144
   Leu Lys Asp Leu Thr Asn Lys Arg Ala Lys Pro Ala Val His Phe Gly
           35                  40                  45
   ggt aag ttc cgc att atc gac ttt gcg ctg tct aac tgc atc aac tcc       192
   Gly Lys Phe Arg Ile Ile Asp Phe Ala Leu Ser Asn Cys Ile Asn Ser
       50                  55                  60
   ggg atc cgt cgt atg ggc gtg atc acc cag tac cag tcc cac act ctg       240
   Gly Ile Arg Arg Met Gly Val Ile Thr Gln Tyr Gln Ser His Thr Leu
   65                  70                  75                  80
   gtg cag cac att cag cgc ggc tgg tca ttc ttc aat gaa gaa atg aac       288
   Val Gln His Ile Gln Arg Gly Trp Ser Phe Phe Asn Glu Glu Met Asn
                   85                  90                  95
   gag ttt gtc gat ctg ctg cca gca cag cag aga atg aaa ggg gaa aac       336
   Glu Phe Val Asp Leu Leu Pro Ala Gln Gln Arg Met Lys Gly Glu Asn
               100                 105                 110
   tgg tat cgc ggc acc gca gat gcg gtc acc caa aac ctc gac att atc       384
   Trp Tyr Arg Gly Thr Ala Asp Ala Val Thr Gln Asn Leu Asp Ile Ile
           115                 120                 125
   cgc cgt tat aaa gcg gaa tac gtg gtg atc ctg gcg ggc gac cat atc      432
   Arg Arg Tyr Lys Ala Glu Tyr Val Val Ile Leu Ala Gly Asp His Ile
       130                 135                 140
   tac aag caa gac tac tcg cgt atg ctt atc gat cac gtc gaa aaa ggc      480
   Tyr Lys Gln Asp Tyr Ser Arg Met Leu Ile Asp His Val Glu Lys Gly
   145                 150                 155                 160
   gca cgt tgc acc gtt gct tgt atg cca gta ccg att gaa gaa gcc tcc      528
   Ala Arg Cys Thr Val Ala Cys Met Pro Val Pro Ile Glu Glu Ala Ser
                   165                 170                 175
   gca ttt ggc gtt atg gcg gtt gat gag aac gat aaa att atc gaa ttc      576
   Ala Phe Gly Val Met Ala Val Asp Glu Asn Asp Lys Ile Ile Glu Phe
               180                 185                 190
   gtt gaa aaa cct gct aac ccg ccg tca atg ccg aac gat ccg agc aaa      624
   Val Glu Lys Pro Ala Asn Pro Pro Ser Met Pro Asn Asp Pro Ser Lys
           195                 200                 205
   tct ctg gcg agt atg ggt atc tac gtc ttt gac gcc gac tat ctg tat      672
   Ser Leu Ala Ser Met Gly Ile Tyr Val Phe Asp Ala Asp Tyr Leu Tyr
       210                 215                 220
   gaa ctg ctg gaa gaa gac gat cgc gat gag aac tcc agc cac gac ttt      720
   Glu Leu Leu Glu Glu Asp Asp Arg Asp Glu Asn Ser Ser His Asp Phe
   225                 230                 235                 240
   ggc aaa gat ttg att ccc aag atc acc gaa gcc ggt ctg gcc tat gcg      768
   Gly Lys Asp Leu Ile Pro Lys Ile Thr Glu Ala Gly Leu Ala Tyr Ala
                   245                 250                 255
   cac ccg ttc ccg ctc tct tgc gta caa tcc gac ccg gat gcc gag ccg      816
   His Pro Phe Pro Leu Ser Cys Val Gln Ser Asp Pro Asp Ala Glu Pro
               260                 265                 270
   tac tgg cgc gat gtg ggt acg ctg gaa gct tac tgg aaa gcg aac ctc      864
   Tyr Trp Arg Asp Val Gly Thr Leu Glu Ala Tyr Trp Lys Ala Asn Leu
           275                 280                 285
   gat ctg gcc tct gtg gtg ccg gaa ctg gat atg tac gat cgc aat tgg      912
   Asp Leu Ala Ser Val Val Pro Glu Leu Asp Met Tyr Asp Arg Asn Trp
       290                 295                 300
   cca att cgc acc tac aat gaa tca tta ccg cca gcg aaa ttc gtg cag      960
   Pro Ile Arg Thr Tyr Asn Glu Ser Leu Pro Pro Ala Lys Phe Val Gln
   305                 310                 315                 320
   gat cgc tcc ggt agc cac ggg atg acc ctt aac tca ctg gtt tcc ggc     1008
   Asp Arg Ser Gly Ser His Gly Met Thr Leu Asn Ser Leu Val Ser Gly
                   325                 330                 335
   ggt tgt gtg atc tcc ggt tcg gtg gtg gtg cag tcc gtt ctg ttc tcg     1056
   Gly Cys Val Ile Ser Gly Ser Val Val Val Gln Ser Val Leu Phe Ser
               340                 345                 350
   cgc gtt cgc gtg aat tca ttc tgc aac att gat tcc gcc gta ttg tta     1104
   Arg Val Arg Val Asn Ser Phe Cys Asn Ile Asp Ser Ala Val Leu Leu
           355                 360                 365
   ccg gaa gta tgg gta ggt cgc tcg tgc cgt ctg cgc cgc tgc gtc atc      1152
   Pro Glu Val Trp Val Gly Arg Ser Cys Arg Leu Arg Arg Cys Val Ile
       370                 375                 380
   gat cgt gct tgt gtt att ccg gaa ggc atg gtg att ggt gaa aac gca      1200
   Asp Arg Ala Cys Val Ile Pro Glu Gly Met Val Ile Gly Glu Asn Ala
   385                 390                 395                 400
   gag gaa gat gca cgt cgt ttc tat cgt tca gaa gaa ggc atc gtg ctg      1248
   Glu Glu Asp Ala Arg Arg Phe Tyr Arg Ser Glu Glu Gly Ile Val Leu
                   405                 410                 415
   gta acg cgc gaa atg cta cgg aag tta ggg cat aaa cag gag cga taa      1296
   Val Thr Arg Glu Met Leu Arg Lys Leu Gly His Lys Gln Glu Arg
               420                 425                 430
<210> 2
   <211> 431
   <212> PRT
   <213> Escherichia coli
<400> 2
   Met Val Ser Leu Glu Lys Asn Asp His Leu Met Leu Ala Arg Gln Leu
   1               5                   10                  15
   Pro Leu Lys Ser Val Ala Leu Ile Leu Ala Gly Gly Arg Gly Thr Arg
               20                  25                  30
   Leu Lys Asp Leu Thr Asn Lys Arg Ala Lys Pro Ala Val His Phe Gly
           35                  40                  45
   Gly Lys Phe Arg Ile Ile Asp Phe Ala Leu Ser Asn Cys Ile Asn Ser
       50                  55                  60
   Gly Ile Arg Arg Met Gly Val Ile Thr Gln Tyr Gln Ser His Thr Leu
   65                  70                  75                  80
   Val Gln His Ile Gln Arg Gly Trp Ser Phe Phe Asn Glu Glu Met Asn
                   85                  90                  95
   Glu Phe Val Asp Leu Leu Pro Ala Gln Gln Arg Met Lys Gly Glu Asn
               100                 105                 110
   Trp Tyr Arg Gly Thr Ala Asp Ala Val Thr Gln Asn Leu Asp Ile Ile
           115                 120                 125
   Arg Arg Tyr Lys Ala Glu Tyr Val Val Ile Leu Ala Gly Asp His Ile
       130                 135                 140
   Tyr Lys Gln Asp Tyr Ser Arg Met Leu Ile Asp His Val Glu Lys Gly
   145                 150                 155                 160
   Ala Arg Cys Thr Val Ala Cys Met Pro Val Pro Ile Glu Glu Ala Ser
                   165                 170                 175
   Ala Phe Gly Val Met Ala Val Asp Glu Asn Asp Lys Ile Ile Glu Phe
               180                 185                 190
   Val Glu Lys Pro Ala Asn Pro Pro Ser Met Pro Asn Asp Pro Ser Lys
           195                 200                 205
   Ser Leu Ala Ser Met Gly Ile Tyr Val Phe Asp Ala Asp Tyr Leu Tyr
       210                 215                 220
   Glu Leu Leu Glu Glu Asp Asp Arg Asp Glu Asn Ser Ser His Asp Phe
   225                 230                 235                 240
   Gly Lys Asp Leu Ile Pro Lys Ile Thr Glu Ala Gly Leu Ala Tyr Ala
                   245                 250                 255
   His Pro Phe Pro Leu Ser Cys Val Gln Ser Asp Pro Asp Ala Glu Pro
               260                 265                 270
   Tyr Trp Arg Asp Val Gly Thr Leu Glu Ala Tyr Trp Lys Ala Asn Leu
           275                 280                 285
   Asp Leu Ala Ser Val Val Pro Glu Leu Asp Met Tyr Asp Arg Asn Trp
       290                 295                 300
   Pro Ile Arg Thr Tyr Asn Glu Ser Leu Pro Pro Ala Lys Phe Val Gln
   305                 310                 315                 320
   Asp Arg Ser Gly Ser His Gly Met Thr Leu Asn Ser Leu Val Ser Gly
                   325                 330                 335
   Gly Cys Val Ile Ser Gly Ser Val Val Val Gln Ser Val Leu Phe Ser
               340                 345                 350
   Arg Val Arg Val Asn Ser Phe Cys Asn Ile Asp Ser Ala Val Leu Leu
           355                 360                 365
   Pro Glu Val Trp Val Gly Arg Ser Cys Arg Leu Arg Arg Cys Val Ile
       370                 375                 380
   Asp Arg Ala Cys Val Ile Pro Glu Gly Met Val Ile Gly Glu Asn Ala
   385                 390                 395                 400
   Glu Glu Asp Ala Arg Arg Phe Tyr Arg Ser Glu Glu Gly Ile Val Leu
                   405                 410                 415
   Val Thr Arg Glu Met Leu Arg Lys Leu Gly His Lys Gln Glu Arg
               420                 425                 430
<210> 3
   <211> 59
   <212> DNA
   <213> Artificial
<220>
   <223> primer
<400> 3
   gtgtgtgttc cagagatgat aaaaaaggag ttagtctgat gtccggcggt gcttttgcc 59
<210> 4
   <211> 57
   <212> DNA
   <213> Artificial
<220>
   <223> primer
   <400> 4
   cgggaacatc tctgaacata catgtaaaac ctgcatttac gccccgccct gccactc 57
<210> 5
   <211> 23
   <212> DNA
   <213> Artificial
<220>
   <223> primer
   <400> 5
   ataacccagt gattacggct gtc 23
<210> 6
   <211> 21
   <212> DNA
   <213> Artificial
<220>
   <223> primer
<400> 6
   cgatttgtgc tgcgggtaat g 21
<210> 7
   <211> 57
   <212> DNA
   <213> Artificial
<220>
   <223> primer
<400> 7
   atgtccgatc gtatcgatag agacgtgatt aacgcgtagt aagccagtat acactcc 57
<210> 8
   <211> 57
   <212> DNA
   <213> Artificial
<220>
   <223> primer
<400> 8
   ttacaatctc accggatcga tatgccagat atgatcttaa gggcaccaat aactgcc 57
<210> 9
   <211> 21
   <212> DNA
   <213> Artificial
<220>
   <223> primer
<400> 9
   ggggtgacac aataaaacag g 21
<210> 10
   <211> 21
   <212> DNA
   <213> Artificial
<220>
   <223> primer
<400> 10
   tggccccgtt ctatttattg g 21
<210> 11
   <211> 1434
   <212> DNA
   <213> Escherichia coli
<220>
   <221> CDS
   <222> (1)..(1434)
<400> 11
   atg cag gtt tta cat gta tgt tca gag atg ttc ccg ctg ctt aaa acc      48
   ggc ggt ctg gct gat gtt att ggg gca tta ccc gca gca caa atc gca      96
   gac ggc gtt gac gct cgc gta ctg ttg cct gca ttt ccc gat att cgc     144
   cgt ggc gtg acc gat gcg cag gta gta tcc cgt cgt gat acc ttc gcc     192
   gga cat atc acg ctg ttg ttc ggt cat tac aac ggg gtt ggc att tac     240
   ctg att gac gcg ccg cat ctc tat gat cgt ccg gga agc ccg tat cac     288
   gat acc aac tta ttt gcc tat acc gac aac gta ttg cgt ttt gcg ctg     336
   ctg ggg tgg gtt ggg gca gaa atg gcc agc ggg ctt gac cca ttc tgg        384
   cgt cct gat gtg gtg cat gcg cac gac tgg cat gca ggc ctt gcg cct        432
   gcg tat ctg gcg gcg cgc ggg cgt ccg gcg aag tcg gtg ttt act gtg        480
   cac aac ctg gcc tat caa ggc atg ttt tat gca cat cac atg aat gac        528
   atc caa ttg cca tgg tca ttc ttt aat att cat ggg ctg gaa ttc aac        576
   gga caa atc tct ttc ctg aag gcc ggt ctg tac tat gcc gat cac att        624
   acg gcg gtc agt cca acc tac gct cgc gag atc acc gaa ccg cag ttt        672
   gcc tac ggt atg gaa ggt ctg ttg caa cag cgt cac cgt gaa ggg cgt        720
   ctt tcc ggc gta ctg aac ggc gtg gac gag aaa atc tgg agt cca gag        768
   acg gac tta ctg ttg gcc tcg cgt tac acc cgc gat acg ttg gaa gat        816
   aaa gcg gaa aat aag cgc cag tta caa atc gca atg ggg ctt aag gtt        864
   gac gat aaa gtg ccg ctt ttt gca gtg gtg agc cgt ctg acc agc cag        912
   aaa ggt ctc gac ctg gtg ctg gaa gcc tta ccg ggt ctt ctg gag cag        960
   ggc ggg cag ctg gcg cta ctc ggc gcg ggc gat ccg gtg ctg cag gaa       1008
   ggt ttc ctt gcg gcg gca gcg gaa tac ccc ggt cag gtg ggc gtt cag       1056
   att ggc tat cac gaa gca ttt tcg cat cgc att atg ggc ggc gcg gac     1104
   gtc att ctg gtg ccc agc cgt ttt gaa ccg tgc ggc tta acg caa ctt     1152
   tat gga ttg aag tac ggt acg ctg ccg tta gtg cgg cgc acc ggt ggg     1200
   ctt gct gat acg gtt tct gac tgt tct ctt gag aac ctt gca gat ggc     1248
   gtc gcc agt ggg ttt gtc ttt gaa gat agt aat gcc tgg tcg ctg tta     1296
   cgg gct att cga cgt gct ttt gta ctg tgg tcc cgt cct tca ctg tgg     1344
   cgg ttt gtg caa cgt cag gct atg gca atg gat ttt agc tgg cag gtc     1392
   gcg gcg aag tcg tac cgt gag ctt tac tat cgc ttg aaa tag             1434
<210> 12
   <211> 477
   <212> PRT
   <213> Escherichia coli
<400> 12
<210> 13
   <211> 2448
   <212> DNA
   <213> Escherichia coli
<220>
   <221> CDS
   <222> (1)..(2448)
<400> 13
   atg aat gct ccg ttt aca tat tca tcg ccc acg ctt agc gta gaa gct        48
   Met Asn Ala Pro Phe Thr Tyr Ser Ser Pro Thr Leu Ser Val Glu Ala
   1               5                   10                  15
   ctt aag cac tct atc gct tac aag ctg atg ttt acg att gga aag gac        96
   Leu Lys His Ser Ile Ala Tyr Lys Leu Met Phe Thr Ile Gly Lys Asp
               20                  25                  30
   ccg gtc gtc gcc aat aaa cat gaa tgg ctg aac gca acg tta ttt gct       144
   Pro Val Val Ala Asn Lys His Glu Trp Leu Asn Ala Thr Leu Phe Ala
           35                  40                  45
   gtg cgc gat cgt ctc gtg gag cgc tgg tta cgt tca aac cgt gcc cag       192
   Val Arg Asp Arg Leu Val Glu Arg Trp Leu Arg Ser Asn Arg Ala Gln
       50                  55                  60
   ttg tcg caa gaa act cgt cag gtt tac tac ctg tcg atg gag ttt ttg       240
   Leu Ser Gln Glu Thr Arg Gln Val Tyr Tyr Leu Ser Met Glu Phe Leu
   65                  70                  75                  80
   att ggc cgt acg ctc tcc aac gcc atg ttg tcg cta gga att tac gaa       288
   Ile Gly Arg Thr Leu Ser Asn Ala Met Leu Ser Leu Gly Ile Tyr Glu
                   85                  90                  95
   gat gta cag ggc gca ctg gaa gcg atg ggg tta aat ctc gaa gag ctg       336
   Asp Val Gln Gly Ala Leu Glu Ala Met Gly Leu Asn Leu Glu Glu Leu
               100                 105                 110
   att gat gaa gaa aat gac cca ggc ctc ggt aac ggt ggc ctg gga cgt       384
   Ile Asp Glu Glu Asn Asp Pro Gly Leu Gly Asn Gly Gly Leu Gly Arg
           115                 120                 125
   ctg gcg gct tgc ttc ctt gat tct ctg gcg acg tta ggg ttg ccg ggg       432
   Leu Ala Ala Cys Phe Leu Asp Ser Leu Ala Thr Leu Gly Leu Pro Gly
       130                 135                 140
   cgc ggt tac ggc atc cgc tat gac tac ggt atg ttc aag cag aac atc       480
   Arg Gly Tyr Gly Ile Arg Tyr Asp Tyr Gly Met Phe Lys Gln Asn Ile
   145                 150                 155                 160
   gtt aac ggt agc cag aaa gag tcg cca gac tac tgg ctg gaa tac ggt      528
   Val Asn Gly Ser Gln Lys Glu Ser Pro Asp Tyr Trp Leu Glu Tyr Gly
                   165                 170                 175
   aac ccg tgg gaa ttc aaa cgc cac aac acg cgc tat aaa gtc cgt ttt      576
   Asn Pro Trp Glu Phe Lys Arg His Asn Thr Arg Tyr Lys Val Arg Phe
               180                 185                 190
   ggc ggt cgc att cag cag gaa ggt aaa aaa acg cgc tgg att gaa acc      624
   Gly Gly Arg Ile Gln Gln Glu Gly Lys Lys Thr Arg Trp Ile Glu Thr
           195                 200                 205
   gaa gag att ctg gga gtc gct tac gat cag ata atc cct ggt tac gac      672
   Glu Glu Ile Leu Gly Val Ala Tyr Asp Gln Ile Ile Pro Gly Tyr Asp
       210                 215                 220
   acc gac gcg acc aac acg ctg cgt ttg tgg agt gcg caa gcc agt agc      720
   Thr Asp Ala Thr Asn Thr Leu Arg Leu Trp Ser Ala Gln Ala Ser Ser
   225                 230                 235                 240
   gaa att aac ctc ggt aaa ttc aac cag ggt gac tac ttc gcg gca gtg      768
   Glu Ile Asn Leu Gly Lys Phe Asn Gln Gly Asp Tyr Phe Ala Ala Val
                   245                 250                 255
   gaa gat aaa aac cac tcc gag aac gta tct cgc gta ctg tat ccg gat      816
   Glu Asp Lys Asn His Ser Glu Asn Val Ser Arg Val Leu Tyr Pro Asp
               260                 265                 270
   gac tcc acc tac tcc ggg cgt gag ctg cgc ctg cgt cag gaa tac ttc      864
   Asp Ser Thr Tyr Ser Gly Arg Glu Leu Arg Leu Arg Gln Glu Tyr Phe
           275                 280                 285
   ctg gtt tcc tcg acc att cag gac att tta agc cgc cat tat cag ttg      912
   Leu Val Ser Ser Thr Ile Gln Asp Ile Leu Ser Arg His Tyr Gln Leu
       290                 295                 300
   cat aaa acc tac gat aac ctg gcg gat aaa atc gcg att cat ctc aat      960
   His Lys Thr Tyr Asp Asn Leu Ala Asp Lys Ile Ala Ile His Leu Asn
   305                 310                 315                 320
   gat acc cat ccg gta ctg tcg att cct gag atg atg cgt ctg ctg atc     1008
   Asp Thr His Pro Val Leu Ser Ile Pro Glu Met Met Arg Leu Leu Ile
                   325                 330                 335
   gat gag cac caa ttt agc tgg gac gac gcg ttt gag gtg tgt tgt cag     1056
   Asp Glu His Gln Phe Ser Trp Asp Asp Ala Phe Glu Val Cys Cys Gln
               340                 345                 350
   gtc ttc tcc tac act aac cac acg ctg atg agc gag gcg ctg gaa acc     1104
   Val Phe Ser Tyr Thr Asn His Thr Leu Met Ser Glu Ala Leu Glu Thr
           355                 360                 365
   tgg ccg gtt gat atg ctg ggt aaa att ctg ccg cgt cac ctg cag atc     1152
   Trp Pro Val Asp Met Leu Gly Lys Ile Leu Pro Arg His Leu Gln Ile
       370                 375                 380
   atc ttt gaa atc aac gac tat ttc ctg aaa acc ttg cag gaa cag tat     1200
   Ile Phe Glu Ile Asn Asp Tyr Phe Leu Lys Thr Leu Gln Glu Gln Tyr
   385                 390                 395                 400
   ccg aac gat acc gat ctg ctg gga cgg gcg tcg atc att gat gaa tcc      1248
   Pro Asn Asp Thr Asp Leu Leu Gly Arg Ala Ser Ile Ile Asp Glu Ser
                   405                 410                 415
   aac ggt cgt cgt gtg cgt atg gcc tgg ctg gcg gtt gtt gtg agc cac      1296
   Asn Gly Arg Arg Val Arg Met Ala Trp Leu Ala Val Val Val Ser His
               420                 425                 430
   aaa gtt aac ggt gta tcg gaa ctg cac tct aat ctg atg gtg caa tcg      1344
   Lys Val Asn Gly Val Ser Glu Leu His Ser Asn Leu Met Val Gln Ser
           435                 440                 445
   ttg ttt gcc gac ttt gcg aaa atc ttc ccg ggt cgt ttc acc aac gtc      1392
   Leu Phe Ala Asp Phe Ala Lys Ile Phe Pro Gly Arg Phe Thr Asn Val
       450                 455                 460
   acc aac ggt gtg acg ccg cgt cgc tgg ctg gcg gta gcg aac cca tcg      1440
   Thr Asn Gly Val Thr Pro Arg Arg Trp Leu Ala Val Ala Asn Pro Ser
   465                 470                 475                 480
   ctt tca gcc gtg ctg gac gaa cac ctg ggc cgt aac tgg cgc acc gac      1488
   Leu Ser Ala Val Leu Asp Glu His Leu Gly Arg Asn Trp Arg Thr Asp
                   485                 490                 495
   ctt agc ctg ctt aat gag ctg caa caa cac tgt gat ttc cca atg gtt      1536
   Leu Ser Leu Leu Asn Glu Leu Gln Gln His Cys Asp Phe Pro Met Val
               500                 505                 510
   aat cac gct gtg cat cag gcg aag ctg gag aac aaa aag cgt ctg gca      1584
   Asn His Ala Val His Gln Ala Lys Leu Glu Asn Lys Lys Arg Leu Ala
           515                 520                 525
   gag tat atc gcc cag cag ctg aat gtg gtg gtg aat cca aag gcg ttg      1632
   Glu Tyr Ile Ala Gln Gln Leu Asn Val Val Val Asn Pro Lys Ala Leu
       530                 535                 540
   ttc gat gta caa atc aaa cgt att cac gaa tac aaa cgt caa ttg atg      1680
   Phe Asp Val Gln Ile Lys Arg Ile His Glu Tyr Lys Arg Gln Leu Met
   545                 550                 555                 560
   aat gtg ttg cat gtg att acc cgc tat aac cgc atc aag gcc gac ccg      1728
   Asn Val Leu His Val Ile Thr Arg Tyr Asn Arg Ile Lys Ala Asp Pro
                   565                 570                 575
   gat gcg aag tgg gta ccg cgc gtg aat att ttt ggc ggt aag gcg gct      1776
   Asp Ala Lys Trp Val Pro Arg Val Asn Ile Phe Gly Gly Lys Ala Ala
               580                 585                 590
   tcg gcc tat tac atg gcg aag cac att att cat ttg atc aat gac gta      1824
   Ser Ala Tyr Tyr Met Ala Lys His Ile Ile His Leu Ile Asn Asp Val
           595                 600                 605
   gcg aaa gtg atc aac aac gat ccg cag att ggc gat aag ctg aaa gtc      1872
   Ala Lys Val Ile Asn Asn Asp Pro Gln Ile Gly Asp Lys Leu Lys Val
       610                 615                 620
   gtg ttc atc ccg aac tac agc gtt agc ctg gcg cag ttg atc att ccg      1920
   Val Phe Ile Pro Asn Tyr Ser Val Ser Leu Ala Gln Leu Ile Ile Pro
   625                 630                 635                 640
   gcg gca gat ctg tct gaa cag att tcg ctg gca ggg acg gaa gct tcc      1968
   Ala Ala Asp Leu Ser Glu Gln Ile Ser Leu Ala Gly Thr Glu Ala Ser
                   645                 650                 655
   ggc acc agt aac atg aag ttt gcg ctt aac ggt gcg ctg act atc ggt      2016
   Gly Thr Ser Asn Met Lys Phe Ala Leu Asn Gly Ala Leu Thr Ile Gly
               660                 665                 670
   acg ttg gac ggt gcg aat gtc gag atg ctg gat cat gtc ggt gct gac      2064
   Thr Leu Asp Gly Ala Asn Val Glu Met Leu Asp His Val Gly Ala Asp
           675                 680                 685
   aat atc ttt att ttt ggt aac aca gcg gaa gaa gtg gaa gaa ctg cgt      2112
   Asn Ile Phe Ile Phe Gly Asn Thr Ala Glu Glu Val Glu Glu Leu Arg
       690                 695                 700
   cgt cag ggc tac aaa ccg cgt gaa tac tac gag aaa gat gag gag ctg      2160
   Arg Gln Gly Tyr Lys Pro Arg Glu Tyr Tyr Glu Lys Asp Glu Glu Leu
   705                 710                 715                 720
   cat cag gtg ctg acg caa atc ggc agc ggt gta ttc agt ccg gaa gat      2208
   His Gln Val Leu Thr Gln Ile Gly Ser Gly Val Phe Ser Pro Glu Asp
                   725                 730                 735
   ccg ggt cgc tat cgc gat ctg gtt gat tcg ctg atc aac ttc ggc gat      2256
   Pro Gly Arg Tyr Arg Asp Leu Val Asp Ser Leu Ile Asn Phe Gly Asp
               740                 745                 750
   cac tac cag gta ctg gcg gat tat cgc agc tat gtc gat tgt cag gat      2304
   His Tyr Gln Val Leu Ala Asp Tyr Arg Ser Tyr Val Asp Cys Gln Asp
           755                 760                 765
   aaa gtc gat gaa ctc tac gag ctt cag gaa gag tgg acc gca aaa gcg      2352
   Lys Val Asp Glu Leu Tyr Glu Leu Gln Glu Glu Trp Thr Ala Lys Ala
       770                 775                 780
   atg ctg aac att gcc aat atg ggc tac ttc tct tct gac cgt act atc      2400
   Met Leu Asn Ile Ala Asn Met Gly Tyr Phe Ser Ser Asp Arg Thr Ile
   785                 790                 795                 800
   aaa gag tac gcc gat cat atc tgg cat atc gat ccg gtg aga ttg taa      2448
   Lys Glu Tyr Ala Asp His Ile Trp His Ile Asp Pro Val Arg Leu
                   805                 810                 815
<210> 14
   <211> 815
   <212> PRT
   <213> Escherichia coli
<400> 14
   Met Asn Ala Pro Phe Thr Tyr Ser Ser Pro Thr Leu Ser Val Glu Ala
   1               5                   10                  15
   Leu Lys His Ser Ile Ala Tyr Lys Leu Met Phe Thr Ile Gly Lys Asp
               20                  25                  30
   Pro Val Val Ala Asn Lys His Glu Trp Leu Asn Ala Thr Leu Phe Ala
           35                  40                  45
   Val Arg Asp Arg Leu Val Glu Arg Trp Leu Arg Ser Asn Arg Ala Gln
       50                  55                  60
   Leu Ser Gln Glu Thr Arg Gln Val Tyr Tyr Leu Ser Met Glu Phe Leu
   65                  70                  75                  80
   Ile Gly Arg Thr Leu Ser Asn Ala Met Leu Ser Leu Gly Ile Tyr Glu
                   85                  90                  95
   Asp Val Gln Gly Ala Leu Glu Ala Met Gly Leu Asn Leu Glu Glu Leu
               100                 105                 110
   Ile Asp Glu Glu Asn Asp Pro Gly Leu Gly Asn Gly Gly Leu Gly Arg
           115                 120                 125
   Leu Ala Ala Cys Phe Leu Asp Ser Leu Ala Thr Leu Gly Leu Pro Gly
       130                 135                 140
   Arg Gly Tyr Gly Ile Arg Tyr Asp Tyr Gly Met Phe Lys Gln Asn Ile
   145                 150                 155                 160
   Val Asn Gly Ser Gln Lys Glu Ser Pro Asp Tyr Trp Leu Glu Tyr Gly
                   165                 170                 175
   Asn Pro Trp Glu Phe Lys Arg His Asn Thr Arg Tyr Lys Val Arg Phe
               180                 185                 190
   Gly Gly Arg Ile Gln Gln Glu Gly Lys Lys Thr Arg Trp Ile Glu Thr
           195                 200                 205
   Glu Glu Ile Leu Gly Val Ala Tyr Asp Gln Ile Ile Pro Gly Tyr Asp
       210                 215                 220
   Thr Asp Ala Thr Asn Thr Leu Arg Leu Trp Ser Ala Gln Ala Ser Ser
   225                 230                 235                 240
   Glu Ile Asn Leu Gly Lys Phe Asn Gln Gly Asp Tyr Phe Ala Ala Val
                   245                 250                 255
   Glu Asp Lys Asn His Ser Glu Asn Val Ser Arg Val Leu Tyr Pro Asp
               260                 265                 270
   Asp Ser Thr Tyr Ser Gly Arg Glu Leu Arg Leu Arg Gln Glu Tyr Phe
           275                 280                 285
   Leu Val Ser Ser Thr Ile Gln Asp Ile Leu Ser Arg His Tyr Gln Leu
       290                 295                 300
   His Lys Thr Tyr Asp Asn Leu Ala Asp Lys Ile Ala Ile His Leu Asn
   305                 310                 315                 320
   Asp Thr His Pro Val Leu Ser Ile Pro Glu Met Met Arg Leu Leu Ile
                   325                 330                 335
   Asp Glu His Gln Phe Ser Trp Asp Asp Ala Phe Glu Val Cys Cys Gln
               340                 345                 350
   Val Phe Ser Tyr Thr Asn His Thr Leu Met Ser Glu Ala Leu Glu Thr
           355                 360                 365
   Trp Pro Val Asp Met Leu Gly Lys Ile Leu Pro Arg His Leu Gln Ile
       370                 375                 380
   Ile Phe Glu Ile Asn Asp Tyr Phe Leu Lys Thr Leu Gln Glu Gln Tyr
   385                 390                 395                 400
   Pro Asn Asp Thr Asp Leu Leu Gly Arg Ala Ser Ile Ile Asp Glu Ser
                   405                 410                 415
   Asn Gly Arg Arg Val Arg Met Ala Trp Leu Ala Val Val Val Ser His
               420                 425                 430
   Lys Val Asn Gly Val Ser Glu Leu His Ser Asn Leu Met Val Gln Ser
           435                 440                 445
   Leu Phe Ala Asp Phe Ala Lys Ile Phe Pro Gly Arg Phe Thr Asn Val
       450                 455                 460
   Thr Asn Gly Val Thr Pro Arg Arg Trp Leu Ala Val Ala Asn Pro Ser
   465                 470                 475                 480
   Leu Ser Ala Val Leu Asp Glu His Leu Gly Arg Asn Trp Arg Thr Asp
                   485                 490                 495
   Leu Ser Leu Leu Asn Glu Leu Gln Gln His Cys Asp Phe Pro Met Val
               500                 505                 510
   Asn His Ala Val His Gln Ala Lys Leu Glu Asn Lys Lys Arg Leu Ala
           515                 520                 525
   Glu Tyr Ile Ala Gln Gln Leu Asn Val Val Val Asn Pro Lys Ala Leu
       530                 535                 540
   Phe Asp Val Gln Ile Lys Arg Ile His Glu Tyr Lys Arg Gln Leu Met
   545                 550                 555                 560
   Asn Val Leu His Val Ile Thr Arg Tyr Asn Arg Ile Lys Ala Asp Pro
                   565                 570                 575
   Asp Ala Lys Trp Val Pro Arg Val Asn Ile Phe Gly Gly Lys Ala Ala
               580                 585                 590
   Ser Ala Tyr Tyr Met Ala Lys His Ile Ile His Leu Ile Asn Asp Val
           595                 600                 605
   Ala Lys Val Ile Asn Asn Asp Pro Gln Ile Gly Asp Lys Leu Lys Val
       610                 615                 620
   Val Phe Ile Pro Asn Tyr Ser Val Ser Leu Ala Gln Leu Ile Ile Pro
   625                 630                 635                 640
   Ala Ala Asp Leu Ser Glu Gln Ile Ser Leu Ala Gly Thr Glu Ala Ser
                   645                 650                 655
   Gly Thr Ser Asn Met Lys Phe Ala Leu Asn Gly Ala Leu Thr Ile Gly
               660                 665                 670
   Thr Leu Asp Gly Ala Asn Val Glu Met Leu Asp His Val Gly Ala Asp
           675                 680                 685
   Asn Ile Phe Ile Phe Gly Asn Thr Ala Glu Glu Val Glu Glu Leu Arg
       690                 695                 700
   Arg Gln Gly Tyr Lys Pro Arg Glu Tyr Tyr Glu Lys Asp Glu Glu Leu
   705                 710                 715                 720
   His Gln Val Leu Thr Gln Ile Gly Ser Gly Val Phe Ser Pro Glu Asp
                   725                 730                 735
   Pro Gly Arg Tyr Arg Asp Leu Val Asp Ser Leu Ile Asn Phe Gly Asp
               740                 745                 750
   His Tyr Gln Val Leu Ala Asp Tyr Arg Ser Tyr Val Asp Cys Gln Asp
           755                 760                 765
   Lys Val Asp Glu Leu Tyr Glu Leu Gln Glu Glu Trp Thr Ala Lys Ala
       770                 775                 780
   Met Leu Asn Ile Ala Asn Met Gly Tyr Phe Ser Ser Asp Arg Thr Ile
   785                 790                 795                 800
   Lys Glu Tyr Ala Asp His Ile Trp His Ile Asp Pro Val Arg Leu
                   805                 810                 815
<210> 15
   <211> 1974
   <212> DNA
   <213> Escherichia coli
<220>
   <221> CDS
   <222> (1)..(1974)
<400> 15
   atg aca caa ctc gcc att ggc aaa ccc gct ccc ctc ggc gcg cat tac        48
   Met Thr Gln Leu Ala Ile Gly Lys Pro Ala Pro Leu Gly Ala His Tyr
   1               5                   10                  15
   gac ggt cag ggc gtc aac ttc aca ctt ttc tcc gct cat gcc gag cgg        96
   Asp Gly Gln Gly Val Asn Phe Thr Leu Phe Ser Ala His Ala Glu Arg
               20                  25                  30
   gta gaa ctg tgt gtc ttt gac gcc aat ggc cag gaa cat cgc tat gac       144
   Val Glu Leu Cys Val Phe Asp Ala Asn Gly Gln Glu His Arg Tyr Asp
           35                  40                  45
   ttg cca ggg cac agt ggc gac att tgg cac ggt tat ctg ccg gat gcg       192
   Leu Pro Gly His Ser Gly Asp Ile Trp His Gly Tyr Leu Pro Asp Ala
       50                  55                  60
   cgc ccg ggt ttg cgt tat ggt tat cgc gtt cat ggc ccc tgg caa ccc       240
   Arg Pro Gly Leu Arg Tyr Gly Tyr Arg Val His Gly Pro Trp Gln Pro
   65                  70                  75                  80
   gcc gag ggg cat cgc ttt aac ccg gcg aag ttg ttg att gat cct tgc       288
   Ala Glu Gly His Arg Phe Asn Pro Ala Lys Leu Leu Ile Asp Pro Cys
                   85                  90                  95
   gcg cgg caa att gac ggg gag ttt aaa gat aac ccg ctg ctg cac gcc      336
   Ala Arg Gln Ile Asp Gly Glu Phe Lys Asp Asn Pro Leu Leu His Ala
               100                 105                 110
   ggt cat aat gaa cct gac tat cgc gac aac gcc gcc att gcg ccg aaa      384
   Gly His Asn Glu Pro Asp Tyr Arg Asp Asn Ala Ala Ile Ala Pro Lys
           115                 120                 125
   tgc gta gtg gtg gtt gat cac tat gac tgg gaa gat gat gcc ccg ccg      432
   Cys Val Val Val Val Asp His Tyr Asp Trp Glu Asp Asp Ala Pro Pro
       130                 135                 140
   cgc acg ccg tgg ggc agc acc atc att tat gaa gcc cat gtc aaa gga      480
   Arg Thr Pro Trp Gly Ser Thr Ile Ile Tyr Glu Ala His Val Lys Gly
   145                 150                 155                 160
   tta acg tac ttg cac ccg gag atc ccg gtc gag atc cgt ggc act tat      528
   Leu Thr Tyr Leu His Pro Glu Ile Pro Val Glu Ile Arg Gly Thr Tyr
                   165                 170                 175
   aaa gcc ctc ggg cat ccg gtg atg atc aac tat ttg aaa caa ttg ggc      576
   Lys Ala Leu Gly His Pro Val Met Ile Asn Tyr Leu Lys Gln Leu Gly
               180                 185                 190
   att acc gcg ctg gaa ctg ctg cca gtg gcg cag ttt gcc agt gaa cca      624
   Ile Thr Ala Leu Glu Leu Leu Pro Val Ala Gln Phe Ala Ser Glu Pro
           195                 200                 205
   cgt ctg caa cgc atg ggg cta agt aac tac tgg ggt tac aac ccg gtg      672
   Arg Leu Gln Arg Met Gly Leu Ser Asn Tyr Trp Gly Tyr Asn Pro Val
       210                 215                 220
   gcg atg ttt gcg ctg cat ccg gcg tat gcc tgc tcg cca gaa acg gcg      720
   Ala Met Phe Ala Leu His Pro Ala Tyr Ala Cys Ser Pro Glu Thr Ala
   225                 230                 235                 240
   ctg gat gag ttt cgc gat gca atc aaa gca ctg cat aaa gcg ggt atc      768
   Leu Asp Glu Phe Arg Asp Ala Ile Lys Ala Leu His Lys Ala Gly Ile
                   245                 250                 255
   gaa gtc att ctt gat atc gtg ctc aac cat agt gcg gaa ctg gac ctc      816
   Glu Val Ile Leu Asp Ile Val Leu Asn His Ser Ala Glu Leu Asp Leu
               260                 265                 270
   gac ggc ccg tta ttc tcg ctg cgt ggg atc gat aac cgt agc tat tat      864
   Asp Gly Pro Leu Phe Ser Leu Arg Gly Ile Asp Asn Arg Ser Tyr Tyr
           275                 280                 285
   tgg ata aga gaa gac ggc gat tat cac aac tgg acc ggt tgc ggc aac      912
   Trp Ile Arg Glu Asp Gly Asp Tyr His Asn Trp Thr Gly Cys Gly Asn
       290                 295                 300
   acg ctc aat ttg agt cat ccg gcg gtg gtg gat tat gcc agc gcc tgc      960
   Thr Leu Asn Leu Ser His Pro Ala Val Val Asp Tyr Ala Ser Ala Cys
   305                 310                 315                 320
   ctg cgt tat tgg gta gaa acc tgc cac gtc gat ggt ttc cgc ttt gat     1008
   Leu Arg Tyr Trp Val Glu Thr Cys His Val Asp Gly Phe Arg Phe Asp
                   325                 330                 335
   ctg gcg gca gtc atg ggc cgt acg cca gag ttc cgt cag gat gcg ccg      1056
   Leu Ala Ala Val Met Gly Arg Thr Pro Glu Phe Arg Gln Asp Ala Pro
               340                 345                 350
   ttg ttt acc gct atc cag aac tgc ccg gtg ctc tcg cag gtg aag tta      1104
   Leu Phe Thr Ala Ile Gln Asn Cys Pro Val Leu Ser Gln Val Lys Leu
           355                 360                 365
   att gct gaa ccg tgg gat atc gct cct ggt ggt tat cag gtg gga aat      1152
   Ile Ala Glu Pro Trp Asp Ile Ala Pro Gly Gly Tyr Gln Val Gly Asn
       370                 375                 380
   ttc ccg ccg ctg ttt gcc gag tgg aac gat cat ttc cgc gat gct gcc      1200
   Phe Pro Pro Leu Phe Ala Glu Trp Asn Asp His Phe Arg Asp Ala Ala
   385                 390                 395                 400
   cgt cgt ttc tgg cta cat tat gat ttg cct ctg ggg gcg ttt gcc ggg      1248
   Arg Arg Phe Trp Leu His Tyr Asp Leu Pro Leu Gly Ala Phe Ala Gly
                   405                 410                 415
   cgt ttt gct gcc tcc agc gat gtt ttt aaa cgt aat ggt cgt ctg ccg      1296
   Arg Phe Ala Ala Ser Ser Asp Val Phe Lys Arg Asn Gly Arg Leu Pro
               420                 425                 430
   agt gcc gcg att aat ctc gtc acc gcg cat gac ggt ttt acg ctt cgc      1344
   Ser Ala Ala Ile Asn Leu Val Thr Ala His Asp Gly Phe Thr Leu Arg
           435                 440                 445
   gac tgc gtt tgc ttc aac cat aaa cac aat gaa gca aac gga gaa gaa      1392
   Asp Cys Val Cys Phe Asn His Lys His Asn Glu Ala Asn Gly Glu Glu
       450                 455                 460
   aat cgc gac ggg acc aac aac aat tac agt aac aat cat ggt aaa gaa      1440
   Asn Arg Asp Gly Thr Asn Asn Asn Tyr Ser Asn Asn His Gly Lys Glu
   465                 470                 475                 480
   ggg tta ggc ggt tct ctt gac ctg gtt gaa cgg cgg cgc gac agc att      1488
   Gly Leu Gly Gly Ser Leu Asp Leu Val Glu Arg Arg Arg Asp Ser Ile
                   485                 490                 495
   cac gcc ctg tta aca acg ttg ttg ctc tcc cag ggt acg ccg atg tta      1536
   His Ala Leu Leu Thr Thr Leu Leu Leu Ser Gln Gly Thr Pro Met Leu
               500                 505                 510
   ctg gcc ggt gac gaa cat ggt cac agc cag cat ggc aat aac aat gcc      1584
   Leu Ala Gly Asp Glu His Gly His Ser Gln His Gly Asn Asn Asn Ala
           515                 520                 525
   tac tgt cag gat aac caa tta acc tgg ttg gac tgg tcg cag gca agc      1632
   Tyr Cys Gln Asp Asn Gln Leu Thr Trp Leu Asp Trp Ser Gln Ala Ser
       530                 535                 540
   agt ggt tta acc gca ttt acc gcc gcg tta atc cat ctg cgc aag cgc      1680
   Ser Gly Leu Thr Ala Phe Thr Ala Ala Leu Ile His Leu Arg Lys Arg
   545                 550                 555                 560
   att ccc gct ttg gtg gag aat cgc tgg tgg gaa gaa ggc gac ggc aat      1728
   Ile Pro Ala Leu Val Glu Asn Arg Trp Trp Glu Glu Gly Asp Gly Asn
                   565                 570                 575
   gtc cgt tgg cta aat cga tat gct caa cct tta agc acg gat gag tgg      1776
   Val Arg Trp Leu Asn Arg Tyr Ala Gln Pro Leu Ser Thr Asp Glu Trp
               580                 585                 590
   caa aac ggg ccg aaa cag ctg caa att ctg ctc tcg gat cgc ttt ttg      1824
   Gln Asn Gly Pro Lys Gln Leu Gln Ile Leu Leu Ser Asp Arg Phe Leu
           595                 600                 605
   atc gca att aac gcc acg ctt gag gta aca gag att gtt tta cct gct      1872
   Ile Ala Ile Asn Ala Thr Leu Glu Val Thr Glu Ile Val Leu Pro Ala
       610                 615                 620
   ggg gag tgg cac gcc att ccc cca ttc gct gga gag gat aac cca gtg      1920
   Gly Glu Trp His Ala Ile Pro Pro Phe Ala Gly Glu Asp Asn Pro Val
   625                 630                 635                 640
   att acg gct gtc tgg cag gga cct gca cac gga ttg tgt gtg ttc cag      1968
   Ile Thr Ala Val Trp Gln Gly Pro Ala His Gly Leu Cys Val Phe Gln
                   645                 650                 655
   aga tga                                                              1974
   Arg
<210> 16
   <211> 657
   <212> PRT
   <213> Escherichia coli
<400> 16
   Met Thr Gln Leu Ala Ile Gly Lys Pro Ala Pro Leu Gly Ala His Tyr
   1               5                   10                  15
   Asp Gly Gln Gly Val Asn Phe Thr Leu Phe Ser Ala His Ala Glu Arg
               20                  25                  30
   Val Glu Leu Cys Val Phe Asp Ala Asn Gly Gln Glu His Arg Tyr Asp
           35                  40                  45
   Leu Pro Gly His Ser Gly Asp Ile Trp His Gly Tyr Leu Pro Asp Ala
       50                  55                  60
   Arg Pro Gly Leu Arg Tyr Gly Tyr Arg Val His Gly Pro Trp Gln Pro
   65                  70                  75                  80
   Ala Glu Gly His Arg Phe Asn Pro Ala Lys Leu Leu Ile Asp Pro Cys
                   85                  90                  95
   Ala Arg Gln Ile Asp Gly Glu Phe Lys Asp Asn Pro Leu Leu His Ala
               100                 105                 110
   Gly His Asn Glu Pro Asp Tyr Arg Asp Asn Ala Ala Ile Ala Pro Lys
           115                 120                 125
   Cys Val Val Val Val Asp His Tyr Asp Trp Glu Asp Asp Ala Pro Pro
       130                 135                 140
   Arg Thr Pro Trp Gly Ser Thr Ile Ile Tyr Glu Ala His Val Lys Gly
   145                 150                 155                 160
   Leu Thr Tyr Leu His Pro Glu Ile Pro Val Glu Ile Arg Gly Thr Tyr
   165 170 175
   Lys Ala Leu Gly His Pro Val Met Ile Asn Tyr Leu Lys Gln Leu Gly
   180 185 190
   Ile Thr Ala Leu Glu Leu Leu Pro Val Ala Gln Phe Ala Ser Glu Pro
   195 200 205
   Arg Leu Gln Arg Met Gly Leu Ser Asn Tyr Trp Gly Tyr Asn Pro Val
   210 215 220
   Ala Met Phe Ala Leu His Pro Ala Tyr Ala Cys Ser Pro Glu Thr Ala
   225 230 235 240
   Leu Asp Glu Phe Arg Asp Ala Ile Lys Ala Leu His Lys Ala Gly Ile
   245 250 255
   Glu Val Ile Leu Asp Ile Val Leu Asn His Ser Ala Glu Leu Asp Leu
   260 265 270
   Asp Gly Pro Leu Phe Ser Leu Arg Gly Ile Asp Asn Arg Ser Tyr Tyr
   275 280 285
   Trp Ile Arg Glu Asp Gly Asp Tyr His Asn Trp Thr Gly Cys Gly Asn
   290 295 300
   Thr Leu Asn Leu Ser His Pro Ala Val Val Asp Tyr Ala Ser Ala Cys
   305 310 315 320
   Leu Arg Tyr Trp Val Glu Thr Cys His Val Asp Gly Phe Arg Phe Asp
   325 330 335
   Leu Ala Ala Val Met Gly Arg Thr Pro Glu Phe Arg Gln Asp Ala Pro
   340 345 350
   Leu Phe Thr Ala Ile Gln Asn Cys Pro Val Leu Ser Gln Val Lys Leu
   355 360 365
   Ile Ala Glu Pro Trp Asp Ile Ala Pro Gly Gly Tyr Gln Val Gly Asn
   370 375 380
   Phe Pro Pro Leu Phe Ala Glu Trp Asn Asp His Phe Arg Asp Ala Ala
   385 390 395 400
   Arg Arg Phe Trp Leu His Tyr Asp Leu Pro Leu Gly Ala Phe Ala Gly
   405 410 415
   Arg Phe Ala Ala Ser Ser Asp Val Phe Lys Arg Asn Gly Arg Leu Pro
   420 425 430
   Ser Ala Ala Ile Asn Leu Val Thr Ala His Asp Gly Phe Thr Leu Arg
   435 440 445
   Asp Cys Val Cys Phe Asn His Lys His Asn Glu Ala Asn Gly Glu Glu
   450 455 460
   Asn Arg Asp Gly Thr Asn Asn Asn Tyr Ser Asn Asn His Gly Lys Glu
   465 470 475 480
   Gly Leu Gly Gly Ser Leu Asp Leu Val Glu Arg Arg Arg Asp Ser Ile
                   485                 490                 495
   His Ala Leu Leu Thr Thr Leu Leu Leu Ser Gln Gly Thr Pro Met Leu
               500                 505                 510
   Leu Ala Gly Asp Glu His Gly His Ser Gln His Gly Asn Asn Asn Ala
           515                 520                 525
   Tyr Cys Gln Asp Asn Gln Leu Thr Trp Leu Asp Trp Ser Gln Ala Ser
       530                 535                 540
   Ser Gly Leu Thr Ala Phe Thr Ala Ala Leu Ile His Leu Arg Lys Arg
   545                 550                 555                 560
   Ile Pro Ala Leu Val Glu Asn Arg Trp Trp Glu Glu Gly Asp Gly Asn
                   565                 570                 575
   Val Arg Trp Leu Asn Arg Tyr Ala Gln Pro Leu Ser Thr Asp Glu Trp
               580                 585                 590
   Gln Asn Gly Pro Lys Gln Leu Gln Ile Leu Leu Ser Asp Arg Phe Leu
           595                 600                 605
   Ile Ala Ile Asn Ala Thr Leu Glu Val Thr Glu Ile Val Leu Pro Ala
       610                 615                 620
   Gly Glu Trp His Ala Ile Pro Pro Phe Ala Gly Glu Asp Asn Pro Val
   625                 630                 635                 640
   Ile Thr Ala Val Trp Gln Gly Pro Ala His Gly Leu Cys Val Phe Gln
                   645                 650                 655
   Arg
<210>- 17
   <211> 2187
   <212> DNA
   <213> Escherichia coli
<220>
   <221> CDS
   <222> (1)..(2187)
<400> 17
<210> 18
   <211> 728
   <212> PRT
   <213> Escherichia coli
<400> 18
   Met Ser Asp Arg Ile Asp Arg Asp Val Ile Asn Ala Leu Ile Ala Gly
   1               5                   10                  15
   His Phe Ala Asp Pro Phe Ser Val Leu Gly Met His Lys Thr Thr Ala
   20 25 30
   Gly Leu Glu Val Arg Ala Leu Leu Pro Asp Ala Thr Asp Val Trp Val
   35 40 45
   Ile Glu Pro Lys Thr Gly Arg Lys Leu Ala Lys Leu Glu Cys Leu Asp
   50 55 60
   Ser Arg Gly Phe Phe Ser Gly Val Ile Pro Arg Arg Lys Asn Phe Phe
   65 70 75 80
   Arg Tyr Gln Leu Ala Val Val Trp His Gly Gln Gln Asn Leu Ile Asp
   85 90 95
   Asp Pro Tyr Arg Phe Gly Pro Leu Ile Gln Glu Met Asp Ala Trp Leu
   100 105 110
   Leu Ser Glu Gly Thr His Leu Arg Pro Tyr Glu Thr Leu Gly Ala His
   115 120 125
   Ala Asp Thr Met Asp Gly Val Thr Gly Thr Arg Phe Ser Val Trp Ala
   130 135 140
   Pro Asn Ala Arg Arg Val Ser Val Val Gly Gln Phe Asn Tyr Trp Asp
   145 150 155 160
   Gly Arg Arg His Pro Met Arg Leu Arg Lys Glu Ser Gly Ile Trp Glu
   165 170 175
   Leu Phe Ile Pro Gly Ala His Asn Gly Gln Leu Tyr Lys Tyr Glu Met
   180 185 190
   Ile Asp Ala Asn Gly Asn Leu Arg Leu Lys Ser Asp Pro Tyr Ala Phe
   195 200 205
   Glu Ala Gln Met Arg Pro Glu Thr Ala Ser Leu Ile Cys Gly Leu Pro
   210 215 220
   Glu Lys Val Val Gln Thr Glu Glu Arg Lys Lys Ala Asn Gln Phe Asp
   225 230 235 240
   Ala Pro Ile Ser Ile Tyr Glu Val His Leu Gly Ser Trp Arg Arg His
   245 250 255
   Thr Asp Asn Asn Phe Trp Leu Ser Tyr Arg Glu Leu Ala Asp Gln Leu
   260 265 270
   Val Pro Tyr Ala Lys Trp Met Gly Phe Thr His Leu Glu Leu Leu Pro
   275 280 285
   Ile Asn Glu His Pro Phe Asp Gly Ser Trp Gly Tyr Gln Pro Thr Gly
   290 295 300
   Leu Tyr Ala Pro Thr Arg Arg Phe Gly Thr Arg Asp Asp Phe Arg Tyr
   305 310 315 320
   Phe Ile Asp Ala Ala His Ala Ala Gly Leu Asn Val Ile Leu Asp Trp
   325 330 335
   Val Pro Gly His Phe Pro Thr Asp Asp Phe Ala Leu Ala Glu Phe Asp
               340                 345                 350
   Gly Thr Asn Leu Tyr Glu His Ser Asp Pro Arg Glu Gly Tyr His Gln
           355                 360                 365
   Asp Trp Asn Thr Leu Ile Tyr Asn Tyr Gly Arg Arg Glu Val Ser Asn
       370                 375                 380
   Phe Leu Val Gly Asn Ala Leu Tyr Trp Ile Glu Arg Phe Gly Ile Asp
   385                 390                 395                 400
   Ala Leu Arg Val Asp Ala Val Ala Ser Met Ile Tyr Arg Asp Tyr Ser
                   405                 410                 415
   Arg Lys Glu Gly Glu Trp Ile Pro Asn Glu Phe Gly Gly Arg Glu Asn
               420                 425                 430
   Leu Glu Ala Ile Glu Phe Leu Arg Asn Thr Asn Arg Ile Leu Gly Glu
           435                 440                 445
   Gln Val Ser Gly Ala Val Thr Met Ala Glu Glu Ser Thr Asp Phe Pro
       450                 455                 460
   Gly Val Ser Arg Pro Gln Asp Met Gly Gly Leu Gly Phe Trp Tyr Lys
   465                 470                 475                 480
   Trp Asn Leu Gly Trp Met His Asp Thr Leu Asp Tyr Met Lys Leu Asp
                   485                 490                 495
   Pro Val Tyr Arg Gln Tyr His His Asp Lys Leu Thr Phe Gly Ile Leu
               500                 505                 510
   Tyr Asn Tyr Thr Glu Asn Phe Val Leu Pro Leu Ser His Asp Glu Val
           515                 520                 525
   Val His Gly Lys Lys Ser Ile Leu Asp Arg Met Pro Gly Asp Ala Trp
       530                 535                 540
   Gln Lys Phe Ala Asn Leu Arg Ala Tyr Tyr Gly Trp Met Trp Ala Phe
   545                 550                 555                 560
   Pro Gly Lys Lys Leu Leu Phe Met Gly Asn Glu Phe Ala Gln Gly Arg
                   565                 570                 575
   Glu Trp Asn His Asp Ala Ser Leu Asp Trp His Leu Leu Glu Gly Gly
               580                 585                 590
   Asp Asn Trp His His Gly Val Gln Arg Leu Val Arg Asp Leu Asn Leu
           595                 600                 605
   Thr Tyr Arg His His Lys Ala Met His Glu Leu Asp Phe Asp Pro Tyr
       610                 615                 620
   Gly Phe Glu Trp Leu Val Val Asp Asp Lys Glu Arg Ser Val Leu Ile
   625                 630                 635                 640
   Phe Val Arg Arg Asp Lys Glu Gly Asn Glu Ile Ile Val Ala Ser Asn
                   645                 650                 655
   Phe Thr Pro Val Pro Arg His Asp Tyr Arg Phe Gly Ile Asn Gln Pro
               660                 665                 670
   Gly Lys Trp Arg Glu Ile Leu Asn Thr Asp Ser Met His Tyr His Gly
           675                 680                 685
   Ser Asn Ala Gly Asn Gly Gly Thr Val His Ser Asp Glu Ile Ala Ser
       690                 695                 700
   His Gly Arg Gln His Ser Leu Ser Leu Thr Leu Pro Pro Leu Ala Thr
   705                 710                 715                 720
   Ile Trp Leu Val Arg Glu Ala Glu
                   725
<210> 19
   <211> 201
   <212> DNA
   <213> Escherichia coli
<220>
   <221> CDS
   <222> (1)..(201)
<400> 19
   atg gat cat agt ctt aat tct tta aat aat ttc gat ttc ctg gcg cgt       48
   Met Asp His Ser Leu Asn Ser Leu Asn Asn Phe Asp Phe Leu Ala Arg
   1               5                   10                  15
   agt ttt gcc aga atg cac gca gaa ggt cgc ccg gtc gat att ctg gcc       96
   Ser Phe Ala Arg Met His Ala Glu Gly Arg Pro Val Asp Ile Leu Ala
               20                  25                  30
   gtt act ggt aac atg gat gaa gaa cat aga acc tgg ttt tgc gca cgt      144
   Val Thr Gly Asn Met Asp Glu Glu His Arg Thr Trp Phe Cys Ala Arg
           35                  40                  45
   tat gcc tgg tat tgt caa cag atg atg cag gca aga gag ctg gag tta      192
   Tyr Ala Trp Tyr Cys Gln Gln Met Met Gln Ala Arg Glu Leu Glu Leu
       50                  55                  60
   gag cac tga                                                          201
   Glu His
   65
<210> 20
   <211> 66
   <212> PRT
   <213> Escherichia coli
<400> 20
   Met Asp His Ser Leu Asn Ser Leu Asn Asn Phe Asp Phe Leu Ala Arg
   1               5                   10                  15
   Ser Phe Ala Arg Met His Ala Glu Gly Arg Pro Val Asp Ile Leu Ala
               20                  25                  30
   Val Thr Gly Asn Met Asp Glu Glu His Arg Thr Trp Phe Cys Ala Arg
           35                  40                  45
   Tyr Ala Trp Tyr Cys Gln Gln Met Met Gln Ala Arg Glu Leu Glu Leu
       50                  55                  60
   Glu His
   65